(19) Europäisches Patentamt
European Patent Office
Office européen des brevets

(11) **EP 2 067 467 A2**

(12) **EUROPEAN PATENT APPLICATION**

(43) Date of publication:
**10.06.2009 Bulletin 2009/24**

(21) Application number: **08253011.4**

(22) Date of filing: **12.09.2008**

(51) Int Cl.:
*A61K 8/84* (2006.01)    *A61Q 5/06* (2006.01)
*A61Q 5/12* (2006.01)    *A61K 8/36* (2006.01)

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **14.09.2007 US 855827**
**14.09.2007 US 855835**
**14.09.2007 US 855843**
**14.09.2007 US 855853**
**14.09.2007 US 855861**
**14.09.2007 US 855869**

(71) Applicant: **L'Oreal**
**75008 Paris (FR)**

(72) Inventors:
• **Nguyen, Nghi Van.**
  **Edison, New Jersey 08820 (US)**
• **Cannell, David.W.**
  **Plainfield, New Jersey 07060 (US)**
• **Hashimoto, Sawa.**
  **Westfield, New Jersey 07090 (US)**
• **Espino, Cynthia.**
  **Princetown, New Jersey 08540 (US)**

(74) Representative: **Rutherford, Jodie**
  **Frank B. Dehn & Co.**
  **St Bride's House**
  **10 Salisbury Square**
  **London**
  **EC4Y 8JD (GB)**

(54) **Compositions and methods for treating keratinous substrates**

(57) The disclosure relates to compositions and methods of using the compositions to treat keratinous substrates. The compositions provide a water resistant and non-transferable protective barrier on the substrate. The compositions contain at least one polyamine, at least one acid, at least one water-insoluble ingredient, solvent and optionally at least one auxiliary ingredient. The methods for treating keratinous substrates involve contacting the keratinous substrates with the compositions of the disclosure.

EP 2 067 467 A2

Description

TECHNICAL FIELD

The disclosure relates to compositions and methods for treating keratinous substrates. The compositions and methods provide a water resistant and non-transferable protective barrier on keratinous substrates imparting the substrates with improved properties. The disclosure relates to compositions and methods for conditioning, shaping, styling and protecting hair. The compositions and methods provide a water resistant and non-transferable protective barrier on hair which conditions hair and protects the hair from adverse environmental conditions. The compositions and methods improve the performance of hair shaping and styling formulations. The compositions and methods provide a water resistant and non-transferable protective barrier on hair that inhibits color fading in both dyed and naturally colored hair. In addition, the disclosure relates to compositions and methods for coloring hair. The compositions and methods also improve the shine of hair.

BACKGROUND OF THE DISCLOSURE

When keratinous substrates are exposed to environmental conditions, the substrates can lose many of their desirable properties. For example, hair can lose its shine, it can become unmanageable, it can lose its color and it can become brittle. One method of maintaining these desirable properties is to provide a protective barrier on keratinous substrates like hair. For example, under low humidity conditions, hair can dry out and dried-out hair tends to be less shiny and more brittle. A protective moisture barrier on the hair will help to keep moisture in the hair allowing hair to keep its shine. Conversely, under high humidity conditions, hair tends to absorb water causing hair to lose its shape and become unmanageable and unattractive. A protective moisture barrier on the hair will help keep moisture out of the hair under high humidity conditions leading to improved manageability. Such a protective barrier can also inhibit color fading in both dyed and naturally colored hair. This protective barrier can be applied to other keratinous substrates such as skin, lips, nails and eyelashes. The protective barrier is also useful in cosmetic applications such as makeup, skin care and sun care products. Such a protective barrier should be water-resistant so that the barrier is not easily removed. In addition, the protective barrier should not be easily transferred from the substrate over time by normal everyday activity. Accordingly, a product that provides a protective barrier to the substrate that also is water resistant and non-transferable would be of benefit to the area of cosmetic products. Typically, hair is colored by application of dyes and/or bleaching agents (coloring agents). For optimum efficiency during the dyeing and/or the bleaching process, the coloring agents should be concentrated as much as possible on the hair fiber. In addition, a barrier to aid in highlighting and lowlighting hair would be useful. Such a barrier would allow coloring agents to selectively color portions of hair while leaving other portions of hair protected and uncolored thus giving highlights or lowlights in selected portions of the hair. Accordingly, a hair coloring composition that aids in the distribution of coloring agents onto the hair fiber would be useful in making more efficient use of hair coloring agents.

BRIEF SUMMARY OF THE DISCLOSURE

The disclosure relates to compositions for treating keratinous substrates and methods of using the compositions to treat hair, skin, eyelashes, nails and lips. The disclosed compositions, which are opaque in nature, provide a water resistant and non-transferable protective barrier on the keratinous substrates imparting desirable properties to the substrate. The methods involve applying the compositions to the substrates. The compositions contain at least one polyamine, at least one acid, at least one water-insoluble ingredient, solvent and optionally at least one auxiliary ingredient. The methods for imparting desirable properties to the keratinous involve contacting the substrate with the compositions of the disclosure. Methods for improving the properties of keratinous substrates are also disclosed. Specifically, methods for improving the shine, condition and manageability of hair are disclosed. In addition, methods of inhibiting color fading in both dyed and naturally colored hair are also disclosed. Finally, methods of making-up eyelashes, making-up lips, making-up facial skin, making-up nails, making-up eyes, protecting skin from UV light damage and chemical damage, reducing the appearance of wrinkles and prolonging the efficacy of an active ingredient on a keratinous substrate are disclosed.

The disclosure relates to compositions and methods of using the compositions for conditioning hair and protecting hair from high and low humidity. The composition provides a water resistant and non-transferable moisture barrier on the hair which keeps moisture in the hair under dry conditions and which keeps moisture out of the hair under humid conditions. The composition contains at least one polyamine, at least one acid, at least one water-insoluble ingredient, at least one conditioning agent, solvent and optionally at least one auxiliary ingredient. The method for conditioning and protecting hair from environmental conditions involves contacting the hair with the composition of the disclosure.

The disclosure relates to compositions and methods of using the compositions for shaping and styling hair.

The composition provides a water resistant and non-transferable moisture barrier on the hair allowing the hair to be more easily shaped and styled. The composition also provides for more effect use of hair styling compositions. The method involves applying the composition to hair to improve the manageability of the hair. The method also involves including a film former in the composition to further improve the shaping and styling characteristics of the hair. The composition contains at least one polyamine, at least one acid, at least one water-insoluble ingredient, at least one film former, solvent and optionally at least one auxiliary ingredient. The method for shaping and styling hair involves contacting the hair with the composition of the disclosure.

The disclosure relates to compositions and methods for coloring hair. The composition provides for a more efficient use of coloring agents by concentrating the color agents more directly onto the hair. The composition contains at least one polyamine, at least one acid, at least one water-insoluble ingredient, at least one hair colorant, solvent, and optionally at least one auxiliary ingredient. The method for coloring hair involves contacting the hair with the composition of the disclosure. The at least one colorant and at least one auxiliary ingredient components may be applied to hair before, during or after the application of the at least one polyamine, the at least one acid and the at least one water-insoluble ingredient which may be applied to hair together.

The disclosure relates to compositions and methods of using the compositions for imparting shine onto hair. The composition provides a water resistant and non-transferable moisture barrier on the hair which keeps moisture in the hair under normal conditions. The composition contains at least one polyamine, at least one acid, at least one water-insoluble ingredient, at least one shine agent, solvent and optionally at least one auxiliary ingredient. The method for conditioning and protecting hair from environmental conditions involves contacting the hair with the composition of the disclosure.

## DETAILED DESCRIPTION OF THE DISCLOSURE

The term "comprising" (and its grammatical variations) as used herein is used in the inclusive sense of "having" or "including" and not in the exclusive sense of "consisting only of'. The terms "a" and "the" as used herein are understood to encompass the plural as well as the singular.

Other than in the operating examples, or where otherwise indicated, all numbers expressing quantities of ingredients and/or reaction conditions are to be understood as being modified in all instances by the term "about".

The term "water-insoluble" means those compounds which are either completely or partially insoluble in water. For example, insoluble or partially insoluble compounds may be those which at a temperature of between 15°C and 25°C per gram of solute there is approximately from 100 to 10000 millilitres of solvent. More preferably there may be from 1000 to 10000 millilitres of solvent or more than 10000 millilitres of solvent per gram of solute. Alternatively viewed, insoluble or partially insoluble means compounds which have a solubility of less than 10g per litre of water, for example less than 5g, 2g, 1g, 0.5g, 0.1g or 0.01g.

"At least one" as used herein means one or more and thus includes individual components as well as mixtures/ combinations. For example, at least one includes 2, 3, 4, 5, 6, 7, 8, 9 or 10 or more components.

"Conditioning" as used herein means imparting to at least one keratinous fiber at least one property chosen from combability, manageability, moisture-retentivity, luster, shine, and softness. The state of conditioning is evaluated by measuring, and comparing, the ease of combability of the treated hair and of the untreated hair in terms of combing work (gm-in).

"Substituted," as used herein, means comprising at least one substituent. Non-limiting examples of substituents include atoms, such as oxygen atoms and nitrogen atoms, as well as functional groups, such as hydroxyl groups, ether groups, alkoxy groups, acyloxyalkyl groups, oxyalkylene groups, polyoxyalkylene groups, carboxylic acid groups, amine groups, acylamino groups, amide groups, halogen containing groups, ester groups, thiol groups, sulphonate groups, thiosulphate groups, siloxane groups, and polysiloxane groups. The substituent(s) may be further substituted.

The at least one polyamine of the disclosure comprises at least two amino groups and typically comprises at least five amino groups and more typically comprises at least ten amino groups. For example the polyamine may comprise 3, 4, 5, 6, 7, 8, 9, 10, 15 or 20 amino groups.

The at least one acid comprises at least one acid group. The at least one acid may also comprise 2, 3, 4, 5, 6, 7, 8, 9, or 10 or more acid groups (a polyacid).

Amino groups include primary amino groups, secondary amino groups and tertiary amino groups and further includes amino groups which are terminal, pendant and intercalated in a skeleton of the at least one polyamine compound.

In an embodiment of the disclosure the composition for treating a keratinous substrate comprises:

at least one polyamine,

at least one acid,

at least one water-insoluble ingredient,

solvent and

optionally at least one auxiliary ingredient

wherein the ratio of the amine number of the at least one polyamine to the acid number of the at least one acid is from about 1:0.5 to about 1:30 and wherein a mixture of the at least one polyamine, the at least one acid, the at least one water-insoluble ingredient and solvent form a mixture that has a contact angle of at least about 66 degrees on glass. The contact angle can be at least 67, 68, 69 or 70 degrees, for example 75 or 80 degrees. Typically, the ratio of the amine number to the acid number is from about 1:0.8 to about 1:20 and more typically from about 1:0.9 to about 1:15 and even more typically from about 1:1 to about 1:10. For example, the ratio can be 1:2, 1:3, 1:4, 1:5, 1:6, 1:7, 1:8 or 1:9.

In another embodiment of the disclosure a composition for treating a hair comprises:

    A. at least one polyamine,

    B. at least one acid,

    C. at least one water-insoluble ingredient,

    D. at least one conditioning agent,

    E. solvent and

    F. optionally at least one auxiliary ingredient

wherein the ratio of the amine number of the at least one polyamine to the acid number of the at least one acid is from about 1:0.5 to about 1:30 and wherein a mixture of the at least one polyamine, the at least one acid, the at least one water-insoluble ingredient and solvent form a mixture that has a contact angle of at least about 66 degrees on glass. Typically, the ratio of the amine number to the acid number is from about 1:0.8 to about 1:20 and more typically from about 1:0.9 to about 1:15 and even more typically from about 1:1 to about 1:10. For example, the ratio can be 1:2, 1:3, 1:4, 1:5, 1:6, 1:7, 1:8 or 1:9.

In yet another embodiment of the disclosure a composition for shaping and styling hair comprises:

    A. at least one polyamine,

    B. at least one acid,

    C. at least one water-insoluble ingredient,

    D. at least one film former,

    E. solvent and

    F. optionally at least one auxiliary ingredient

wherein the ratio of the amine number of the at least one polyamine to the acid number of the at least one acid is from about 1:0.5 to about 1:30 and wherein a mixture of the at least one polyamine, the at least one acid, the at least one water-insoluble ingredient and solvent form a mixture that has a contact angle of at least about 66 degrees on glass. Typically, the ratio of the amine number to the acid number is from about 1:0.8 to about 1:20 and more typically from about 1:0.9 to about 1:15 and even more typically from about 1:1 to about 1:10. For example, the ratio can be 1:2, 1:3, 1:4, 1:5, 1:6, 1:7, 1:8 or 1:9.

The embodiment for shaping and styling hair involves contacting hair with the disclosed composition. In one embodiment, a mixture comprising the at least one polyamine, the at least one acid, the at least one water-insoluble ingredient and solvent and optionally the at least one auxiliary ingredient may be applied to hair followed by application of a mixture comprising the film former component and optionally comprising solvent and the at least one auxiliary ingredient. The hair is then shaped or styled. Hence, products containing the at least one polyamine, the at least one acid, the at least one water-insoluble ingredient and solvent, the optionally at least one auxiliary ingredient and the at least one film former as a combined preparation for simultaneous, separate or sequential use are encompassed.

In another embodiment, a mixture comprising the at least one polyamine, the at least one acid, the at least one water-insoluble ingredient, the at least one film former and solvent and optionally the at least one auxiliary ingredient may be applied to hair. The hair is then shaped or styled.

In another embodiment, a mixture comprising the film former component and optionally solvent, and optionally at least one auxiliary ingredient is applied to hair followed by application of a mixture comprising the at least one polyamine, the at least one acid, the at least one water-insoluble ingredient and solvent and optionally at least one auxiliary ingredient. The hair is then shaped or styled.

Another embodiment of the disclosure involves a composition for coloring hair which comprises:

at least one polyamine,

at least one acid,

at least one water-insoluble ingredient,

at least one hair colorant,

solvent and

optionally at least one auxiliary ingredient

wherein the ratio of the amine number of the at least one polyamine to the acid number of the at least one acid is from about 1:0.5 to about 1:30 and wherein a mixture of the at least one polyamine, the at least one acid, the at least one water-insoluble ingredient and solvent form a mixture that has a contact angle of at least about 66 degrees on glass. Typically, the ratio of the amine number to the acid number is from about 1:0.8 to about 1:20 and more typically from about 1:0.9 to about 1:15 and even more typically from about 1:1 to about 1:10. For example, the ratio can be 1:2, 1:3, 1:4, 1:5, 1:6, 1:7, 1:8 or 1:9.

Another embodiment of the disclosure involves coloring hair with the composition discussed above.

For coloring hair the order of application of the components listed above to the hair is not limited. Each applied component may be alone or with any other component or mixture of components in any order to the hair.

For example, the at least one coloring agent may be applied to hair before the other components, with the other components or after the other components.

Embodiments may include first dying and/or bleaching with the coloring agent then applying the other components of the disclosure to the hair.

Other embodiments may include applying all the components of the disclosure as a single mixture.

Yet other embodiments may include applying components the at least one polyamine, the at least one acid, the at least one water-insoluble ingredient and solvent and optionally components to the hair then coloring the hair with the at least one hair colorant.

The applications described above may be conducted selectively on the hair. That is, some portions of the hair may be first colored with the coloring agent followed by application of the other components and other portions may have all components except the coloring agent applied followed by application of the coloring agent. Again the method of applying the individual components and the order of addition of the components is not limited.

One further embodiment of the disclosure involves a composition for treating a hair which comprises:

A. at least one polyamine,

B. at least one acid,

C. at least one water-insoluble ingredient,

D. at least one shine agent,

E. solvent, and

F. optionally at least one auxiliary ingredient

wherein the ratio of the amine number of the at least one polyamine to the acid number of the at least one acid is from about 1:0.5 to about 1:30 and wherein a mixture of the at least one polyamine, the at least one acid, the at least one water-insoluble ingredient and solvent form a mixture that has a contact angle of at least about 66 degrees on glass. Typically, the ratio of the amine number to the acid number is from about 1:0.8 to about 1:20 and more typically from about 1:0.9 to about 1:15 and even more typically from about 1:1 to about 1:10. For example, the ratio can be 1:2, 1:3, 1:4, 1:5, 1:6, 1:7, 1:8 or 1:9.

Another embodiment of the disclosure involves applying the composition above to hair. This treatment provides hair with improved long lasting shine.

Other embodiments of the disclosure include combining the disclosed composition with hair care products such as shampoos, conditioners, hair gel, hair lotion and hair mousses. The combined composition provides for a modified hair care product with improved performance. Accordingly, such embodiments include a shampoo, conditioner, gel, lotion and mousse containing the disclosed composition.

Another embodiment of the disclosure involves applying the disclosed composition to hair. This treatment protects the hair from adverse environmental conditions.

Other embodiments include combining the disclosed composition with hair care products such as shampoos, conditioners, hair gel, hair lotion and hair mousses. The combined composition provides for a modified hair care product with improved performance. Accordingly, such embodiments include a shampoo, conditioner, gel, lotion and mousse containing the disclosed composition.

Another embodiment of the disclosure involves treating keratinous substrates with the disclosed composition. Such treatments impart desirable properties to the keratinous substrate.

Another embodiment of the disclosure involves treating hair with the disclosed composition to inhibit color fading in dyed and naturally colored hair.

Another embodiment of the disclosure involves treating various keratinous substrates with the disclosed composition. The methods of treating keratinous substrates with the disclosed composition includes making-up eyelashes, making-up lips, making-up facial skin, making-up nails, making-up eyes, protecting skin from UV light damage and chemical damage, reducing the appearance of wrinkles and prolonging the efficacy of an active ingredient on a keratinous substrate.

The at least one polyamine may, for example, be chosen from a polyethyleneimine, a polyvinylamine, an aminated polysaccharide, an amine substituted polyalkylene glycol, an amine substituted polyacrylate crosspolymer, an amine substituted polyacrylate, an amine substituted polymethacrylate, an aminosilicone, a protein, an amine substituted polyester, a polyamino acid, an amodimethicone, a polyalkylamine, diethylene triamine, triethylenetetramine, spermidine, spermine and mixtures thereof.

Non-limiting examples of polyethyleneimine include Lupasol® products commercially available from BASF. Suitable examples of Lupasol® polyethyleneimines include Lupasol® PS, Lupasol® PL, Lupasol® PR8515, Lupasol® G20, Lupasol® G35 as well as Lupasol® SC Polyethyleneimine Reaction Products (such as Lupasol® SC-61B, Lupasol® SC-62J, and Lupasol® SC-86X). Other non-limiting examples of polyethyleneimines which may be used in the composition according to the present invention are the Epomin® products commercially available from Aceto. Suitable examples of Epomin® polyethyleneimines include Epomin® SP-006, Epomin® SP-012, Epomin® SP-018, and Epomin® P-1000. These examples include substituted polyethyleneimines.

Non-limiting examples of polyvinylamines include Lupamines® 9095, 9030, 9010, 5095 and 1595 from BASF.

An example of an amine substituted polyalkylene glycol includes PEG-15 cocopolyamine from Cognis.

An example of an aminosilicone includes Dow Coming® 2-8566 Amino Fluid, an amino functional polydimethylsiloxane fluid from Dow Coming®.

In another embodiment, the at least one polyamine compound is chosen from proteins and protein derivatives. Non-limiting examples of suitable proteins and protein derivatives for use in the present invention include those listed at pages 1701 to 1703 of the C.T.F.A. International Cosmetic Ingredient Dictionary and Handbook, 8th edition, vol. 2, (2000) (incorporated herein by reference). In one embodiment, the at least one polyamine compound is chosen from wheat protein, soy protein, oat protein, collagen, and keratin protein.

In another embodiment, the at least one polyamine compound is chosen from compounds comprising lysine, compounds comprising arginine, compounds comprising histidine, and compounds comprising hydroxylysine. Not limiting examples include chitosan, polyarginine and polylysine.

An example of an amine substituted polyacrylate crosspolymer includes Carbopol® Aqua CC polymer from Lubrizol Advanced Materials, Inc.

In the present disclosure, the at least one polyamine is used in a positive amount up to about 30% by weight, more typically a positive amount up to about 10% by weight, and most typically a positive amount up to about 5% by weight, based on the weight of the composition as a whole. In some embodiments the at least one polyamine ranges from about 0.1% to about 30% by weight based on the weight of the composition. In other embodiments the at least one polyamine ranges from about 0.1 wt % to about 10 wt %, based on the weight of the composition as a whole and in further embodiments the range is from about 0.1 wt % to about 5 wt %.

The at least one acid of the composition may, for example, be chosen from a fatty carboxylic acid, a fatty ether carboxylic acid, a fatty ether phosphoric acid, a fatty phosphoric acid and mixtures thereof. The at least one acid may contain one or 2 or more acid groups (a polyacid).

Non-limiting examples of fatty carboxylic acids includes fatty acids having from about 6 to about 40 carbon atoms corresponding formula (I)

RCOOH          (I)

wherein:

R is a hydrocarbon radical containing from about 6 to about 40 carbon atoms. In addition, R is linear or branched, acyclic or cyclic, saturated or unsaturated, aliphatic or aromatic, substituted or unsubstituted. Typically, R is a linear or branched, acyclic $C_{6-40}$ alkyl or alkenyl group or a $C_{1-40}$ alkyl phenyl group, more typically a $C_{8-22}$ alkyl or alkenyl group

or a $C_{4-18}$ alkyl phenyl group, and even more typically a $C_{12-18}$ alkyl group or alkenyl group or a $C_{6-16}$ alkyl phenyl group.

Suitable fatty acids having from about 6 to about 40 carbon atoms include, but are not limited to the following representatives referred to by their INCI names (INCI: nomenclature for raw materials according to the International Cosmetic Ingredient Dictionary, 10th Edition, published by the Cosmetic, Toiletry and Fragrance Association Inc. (CTFA), Washington D.C., USA): Arachidic Acid, Arachidonic Acid, Beeswax Acid, Capric Acid, Caproic Acid, Caprylic Acid, Coconut Acid, Isostearic Acid, Lauric Acid, Linoleic Acid, Linolenic Acid, Myristic Acid, Oleic Acid, Olive Acid, Palmitic Acid, Rapeseed Acid, Stearic Acid, Behenic Aid, Tallow Acid, Undecanoic Acid, Undecylenic Acid, 18-Methyleicosanoic Acid, Wheat Germ Acid and mixtures thereof.

Typical fatty acids having from about 6 to about 40 carbon atoms include Linoleic Acid, Oleic Acid, Isostearic Acid, and Stearic Acid.

Non-limiting examples of fatty ether carboxylic acid includes compounds corresponding to formula (II):

$$RO(CH_2O]_u(_(CH_2)_xCH(R')(CH_2)_y\ (CH_2)_zO]_v[CH_2CH_2O]_wCH_2COOH \qquad (II)$$

wherein:

R is a hydrocarbon radical containing from about 6 to about 40 carbon atoms;

u, v and w, independently of one another, represent numbers of from 0 to 60;

x, y and z, independently of one another, represent numbers of from 0 to 13;

R' represents hydrogen, alkyl, and

the sum of x+y+z is $\geq$ 0;

Ether carboxylic acids corresponding to formula (II) can be obtained by alkoxylation of alcohols ROH with ethylene oxide as the sole alkoxide or with several alkoxides and subsequent oxidation. The numbers u, v, and w each represent the degree of alkoxylation. Whereas, on a molecular level, the numbers u, v and w and the total degree of alkoxylation can only be integers, including zero, on a macroscopic level they are mean values in the form of broken numbers.

In formula (II), R is linear or branched, acyclic or cyclic, saturated or unsaturated, aliphatic or aromatic, substituted or unsubstituted. Typically, R is a linear or branched, acyclic $C_{6-40}$ alkyl or alkenyl group or a $C_{1-40}$ alkyl phenyl group, more typically a $C_{8-22}$ alkyl or alkenyl group or a $C_{4-18}$ alkyl phenyl group, and even more typically a $C_{12-18}$ alkyl group or alkenyl group or a $C_{6-16}$ alkyl phenyl group; u, v, w, independently of one another, is typically a number from 2 to 20, more typically a number from 3 to 17 and most typically a number from 5 to 15; x, y, z, independently of one another, is typically a number from 2 to 13, more typically a number from 1 to 10 and most typically a number from 0 to 8.

Suitable ether carboxylic acids or ether carboxylates include, but are not limited to, the following representatives referred to by their INCI names (INCI: nomenclature for raw materials according to the International Cosmetic Ingredient Dictionary, 7th Edition, published by the Cosmetic, Toiletry and Fragrance Association Inc. (CTFA), Washington D.C., USA): Butoxynol-5 Carboxylic Acid, Butoxynol-19 Carboxylic Acid, Capryleth-4 Carboxylic Acid, Capryleth-6 Carboxylic Acid, Capryleth-9 Carboxylic Acid, Ceteareth-25 Carboxylic Acid, Coceth-7 Carboxylic Acid, $C_{9-11}$ Pareth-6 Carboxylic Acid, $C_{11-15}$ Pareth-7 Carboxylic Acid, $C_{12-13}$ Pareth-5 Carboxylic Acid, $C_{12-13}$ Pareth-8 Carboxylic Acid, $C_{12-13}$ Pareth-12 Carboxylic Acid, $C_{12-15}$ Pareth-7 Carboxylic Acid, $C_{12-15}$ Pareth-8 Carboxylic Acid, $C_{14-15}$ Pareth-8 Carboxylic Acid, Deceth-7 Carboxylic Acid, Laureth-3 Carboxylic Acid, Laureth-4 Carboxylic Acid, Laureth-5 Carboxylic Acid, Laureth-6 Carboxylic Acid, Laureth-8 Carboxylic Acid, Laureth-10 Carboxylic Acid, Laureth-11 Carboxylic Acid, Laureth-12 Carboxylic Acid, Laureth-13 Carboxylic Acid, Laureth-14 Carboxylic Acid, Laureth-17 Carboxylic Acid, PPG-6-Laureth-6 Carboxylic Acid, PPG-8-Steareth-7 Carboxylic Acid, Myreth-3 Carboxylic Acid, Myreth-5 Carboxylic Acid, Nonoxynol-5 Carboxylic Acid, Nonoxynol-8 Carboxylic Acid, Nonoxynol-10 Carboxylic Acid, Octeth-3 Carboxylic Acid, Octoxynol-20 Carboxylic Acid, Oleth-3 Carboxylic Acid, Oleth-6 Carboxylic Acid, Oleth-10 Carboxylic Acid, PPG-3-Deceth-2 Carboxylic Acid, Capryleth-2 Carboxylic Acid, Ceteth-13 Carboxylic Acid, Deceth-2 Carboxylic Acid, Hexeth-4 Carboxylic Acid, Isosteareth-6 Carboxylic Acid, Isosteareth-11 Carboxylic Acid, Trudeceth-3 Carboxylic Acid, Trideceth-6 Carboxylic Acid, Trideceth-8 Carboxylic Acid, Trideceth-12 Carboxylic Acid, Trideceth-3 Carboxylic Acid, Trideceth-4 Carboxylic Acid, Trideceth-7 Carboxylic Acid, Trideceth-15 Carboxylic Acid, Trideceth-19 Carboxylic Acid, Undeceth-5 Carboxylic Acid and mixtures thereof.

Typical Carboxylic Acids are Oleth-10 Carboxylic Acid, Laureth-5 Carboxylic Acid and Laureth-11 Carboxylic Acid.

Non-limiting examples of fatty phosphoric acids include compounds corresponding to Formula III:

$$R\text{-}O\text{-}P(O)(OH)_2 \qquad (III)$$

wherein:

R is a hydrocarbon radical containing from about 6 to about 40 carbon atoms. In addition, R is linear or branched, acyclic or cyclic, saturated or unsaturated, aliphatic or aromatic, substituted or unsubstituted. Typically, R is a linear or branched acyclic $C_{6-40}$ alkyl or alkenyl group or a $C_{1-40}$ alkyl phenyl group, more typically a $C_{8-22}$ alkyl or alkenyl group or a $C_{4-18}$ alkyl phenyl group and most typically a $C_{12-18}$ alkyl group or alkenyl group or a $C_{6-16}$ alkyl phenyl group.

Typical fatty phosphoric acids include capryl phosphate, caprylyl phosphate, lauryl phosphate, oleyl phosphate, isostearyl phosphate, stearyl phosphate and cetyl phosphate.

Non-limiting examples of fatty ether phosphoric acids compounds corresponding to formulas IV and V:

$$RO[CH_2O]_u[(CH_2)_xCH(R')(CH_2)_y(CH_2)_zO]_v[CH_2CH_2O]_w\text{-PO-(OH)}_2 \qquad \text{Formula IV,}$$

$$\{RO[CH_2O]_u[(CH_2)_xCH(R')(CH_2)_y(CH_2)_zO]_v(CH_2CH_2O]_w\}_2PO\text{-(OH)} \qquad \text{Formula V}$$

and combinations thereof,

wherein:

R is a hydrocarbon radical containing from about 6 to about 40 carbon atoms;

u, v and w, independently of one another, represent numbers of from 0 to 60;

x, y and z, independently of one another, represent numbers of from 0 to 13;

R' represents hydrogen, alkyl, and

the sum of x+y+z being ≥0.

The numbers u, v, and w each represent the degree of alkoxylation. Whereas, on a molecular level, the numbers u, v and w and the total degree of alkoxylation can only be integers, including zero, on a macroscopic level they are mean values in the form of broken numbers.

In formulas IV and V, R is linear of branched, acyclic or cyclic, saturated or unsaturated, aliphatic or aromatic, substituted or unsubstituted, typically a linear or branched, acyclic $C_{6-40}$ alkyl or alkenyl group or a $C_{1-40}$ alkyl phenyl group, more typically a $C_{8-22}$ alkyl or alkenyl group or a $C_{4-18}$ alkyl phenyl group, even more typically a $C_{12-18}$ alkyl group or alkenyl group or a $C_{6-16}$ alkyl phenyl group; u, v, w, independently of one another, is typically a number from 2 to 20, more typically a number from 3 to 17 and most typically a number from 5 to 15; x, y, z, independently of one another, is typically a number from 2 to 13, more typically a number from 1 to 10 and most typically a number from 0 to 8.

Typical fatty ether phosphoric acids include PPG-5-Ceteth-10 phosphate (CRODAFOS SG), Oleth-3 phosphate (CRODAFOS N3 acid), Oleth-10 phosphate (CRODAFOS N10 acid), and a mixture of Ceteth-10 phosphate and Dicetyl phosphate (CRODAFOS CES) all sold by Croda.

Examples of the at least one acid that contain 2 or more acid groups include Acrylates Copolymer, Acrylates/ Octylacrylamide Copolymer, Acrylates/Octylacrylamide/Diphenyl Amodimethicone Copolymer, Octylacrylamide/acrylates/Butylaminoethyl Methacrylate Copolymer, Acrylates/Stearyl Acrylate/Ethylamine Oxide Methacrylate Copolymer, Acrylates/t-Butylacrylamide Copolymer, PEG/PPG-25/25 Dimethicone/Acrylates Copolymer, VA/Butyl Maleate/Isobornyl Acrylate Copolymer, PVM/MA Copolymer, Ethyl ester of PVM/MA Copolymer, Butyl Ester of PVM/MA Copolymer, VA/Crotonates Copolymer, VA/Crotonates/Vinyl Neodecanoate Copolymer, VA/Vinyl Butyl Benzoate/Crotonates Copolymer, Carbomer, Polystyrene sulfonic acid, Terephthalylidene Dicamphor Sulfonic Acid, Phenylbenzimidazole Sulfonic Acid, Polyacrylamidomethylpropane Sulfonic Acid, Dimethicone PEG-7 Phosphate, Dimethicone PEG-8 Phosphate, Dimethicone PEG-10 Phosphate, Dimethicone PEG/PPG-7/4 Phosphate, Dimethicone PEG/PPG-12/4 Phosphate, Lauryl dimethicone PEG-10 Phosphate, Polyperfluoroethoxymethoxy Difluoroethyl PEG Phosphate, Polyperfluoroethoxymethoxy PEG-2 Phosphate, Polyphosphorylcholine Glycol Acrylate, Cocoamphodipropionic Acid, Lauroamphodipropionic Acid, Lauriminodipropionic Acid, Polyacrylic Acid, Polymethacrylic Acid, Polyglutamic acid, Myristoyl Glutamic Acid, Lauroyl Glutamic Acid, Palmitoyl Glutamic Acid, Cocoyl Glutamic Acid.

The at least one acid is present in the composition in a positive amount up to about 50% by weight, typically a positive amount up to about 30% by weight, and more typically a positive amount up to about 15% by weight, based on the weight of the composition as a whole. In other embodiments, the at least one acid is present in the composition in a range of from about 2% to about 50% by weight and in a range from about 5% to about 15% by weight, based on the weight of the composition as a whole.

The at least one water-insoluble ingredient may, for example, be chosen from an oil, a polymer, a fatty ester, a hydrocarbon, a silicone, a wax, a fatty acid (in addition to the fatty acid), salts of fatty acids, a fatty alcohol and mixtures thereof.

Non-limiting examples of oils include plant oil such as olive oil, avocado oil, coconut oil, aloe vera oil, almond oil, castor oil, jojoba oil, peanut oil, sesame oil, hazelnut oil, sunflower oil, colza oil, grapeseed oil, linseed oil and palm oil.

Non-limiting examples of hydrocarbon oils include mineral oil, petrolatum, paraffins, iso-paraffins, aromatic

hydrocarbons and C$_{10-40}$ hydrocarbons which may be aliphatic, aromatic, arylaliphatic or mixtures thereof and the aliphatic hydrocarbons may be straight chain, branched, cyclic or combinations thereof.

Non-limiting examples of silicones include phenyltrimethicone, dimethicone, cyclomethicone, dimethicone copolyol, aminosilicone, laurylmethicone copolyol, cetyl dimethicone, cetyl triethylammonium dimethicone copolyol phthalate, dimethicone copolyol lactate, silicone quaternium-13, stearalkonium dimethicone copolyol phthalate, stearaminopropyl dimethicone and polyorganosiloxanes such as polydimethylsiloxane.

Non-limiting examples of waxes include paraffin wax, beeswax, candelilla wax, carnauba wax, jasmine wax, jojoba wax and mimosa wax.

Non-limiting examples of fatty acids are the same as those described above for the at least one fatty acid described above. This includes carboxylate salts of the fatty acids listed above. The sodium, potassium, ammonium, calcium and magnesium carboxylates of the fatty acids listed above are typical examples of the carboxylate salts of the fatty acids.

Non-limiting example of fatty alcohols include compounds of formula (VI):

R-OH            (VI)

where R is as described above for the at least one fatty acid.

Non-limiting fatty esters include esters formed from the fatty acid of formula (I) and C$_{1-22}$ alcohols and esters formed from the fatty alcohol of formula VI and C$_{1-22}$ carboxylic acids.

In addition, non-limiting specific examples of water-insoluble ingredients includes isopropyl palmitate, capric/caprylic triglyceride, isododecane, polylsobutylene, tocopherol, tocopherol acetate, retinol, retinyl palmitate, 2-oleamido-1,3-octadecanediol, octymethoxy cinnamate, octyl salicylate, 18-Methyleicosanoic Acid and mixtures thereof.

The at least one water-insoluble ingredient is present in the composition in a positive amount up to about 50% by weight, typically a positive amount up to about 30% by weight, and more typically a positive amount up to about 15% by weight based on the weight of the composition as a whole: In other embodiments, the at least one water-insoluble ingredient is present in the composition in an amount from about 0.1 to about 50% by weight and in an amount from about 0.5 to about 15% by weight based on the weight of the composition as a whole.

The at least one conditioning agent may be chosen from amino acids, proteins, extracts, fats, oils, esters, transesters, hydrocarbons, quats, polyquats, zwitterionic surfactants, amphoteric surfactants, alcohols, polyols, humectants, alkanolamides, fatty acids, ketones, and mixtures thereof. The conditioning agent is present in an amount from about 0.001 % to about 50% by weight, based on the weight of the composition. Typically, the conditioning agent is present in an amount from about 0.1% to about 35% by weight, based on the weight of the composition and more typically in an amount from about 1% to about 20% by weight, based on the weight of the composition.

Non-limiting examples of conditioning agents include Arginine, Asparagine, Aspartic Acid, Carnitine, Cocoyl sarcosine, Glycine, Glutamic acid, Histidine, Hydroxyproline, Acetyl Hydroxy praline, Isoleucine, Lysine, Lauroyl Lysine, Lauroyl Sarcosine, Methionine, Phenylalanine, Polylysine, Potassium Cocoyl Glutamate, Proline, Sarcosine, Serine , Rice amino acids, Silk amino acids, Wheat amino aids, Sodium Glutamate, Sodium Lauroyl Glutamate, Sodium PCA, Stearoyl sarcosine, Threonine, Tyrosine, Tryptophan, Valine, Casein, Collagen, Procollagen, Gelatin, Keratin, Glycoproteins, Hydrolyzed wheat protein, Hydrolyzed soy protein, Hydrolyzed oat protein, Hydrolyzed rice protein, Hydrolzed vegetable protein, Hydrolyzed yeast protein, Whey protein , Ginkgo Biloba Nut extract, Salix Alba (Willow) Bark Extract, Morus Alba (Mulberry) Leaf , Behentrimonium Chloride, Behenamidopropyl PG-Dimonium Chloride, Behentrimonium Methosulfate, Cocotrimonium Methosulfate, Olealkonium Chloride, Steartrimonium Chloride, Babassuamidopropalkonium Chloride, Hydroxypropyl Guar , Hydroxypropyltrimonium chloride, Laurdimonium Hydroxypropyl Hydrolyzed Soy Protein, Steardimonium Hydroxypropyl Hydrolyzed Wheat Protein, Quaternium-22, Quaternium-27, Quaternium-87, Polyquaternium-4, Polyquaternium-6, Polyquaternium-10, Polyquaternium-11, Polyquaternium-44, Polyquaternium-67, Silicone Quaterium-8, Amodimethicone, Aminopropyldimethicone, Phenyltrimethicone, Cyclomethicone, Dimethicone, Hexyl Dimethicone, Dilinoleamidopropyl Dimthylamine Dimethicone PEG-7 Phosphate, C26-28 Alkyl Dimethicone, PEG-8 Dimethicone, PPG-12 Dimethicone, Polysilicone-13, Trideceth-9 PG-Amodimethicone, Bis-PEG-12 Dimethicone Beeswax, Capric/Caprylic Triglyceride, Petrolatum, Mineral Oil, Lanolin Oil, Cocos nucifera (Coconut) Oil, Olea Europea (Olive) Fruit Oil , Simmondsia Chinensis (Jojoba) Seed Oil, Prunus Armeniaca (Apricot) Kernel Oil, Crambe Abyssinica Seed Oil, Vegetable Oil, Zea Mays (Corn) Oil, Acetylated Lanolin Alcohol, Cetearyl Isononanoate, Cetearyl Ethylhexanoate, Cetearyl Palmitate, Hydrogenated Olive Oil Hexyl Esters, Triethylhexanoin, Ceramide-3, Caprylyl Glycol, Cetyl Glycol, Glycerin, Panthenol, Phytantriol, Methanediol, Inositol, PPG-35-Buteth-45, PPG-5 Butyl Ether, Cocoamidopropyl Betaine, Coco-Betaine, Cocoamidopropyl Hydroxysultaine, Lauramidopropyl Betaine, Lauryl Betaine, Oleamidopropyl Betaine, Disodium Cocoamphodiacetate, Disodium Cocoamphodipropionate, Disodium Lauroamphodiacetate, Sodium Cocoamphopropionate, Sodium Cocoamphoacetate, Acetamide MEA, Behenamide MEA, Linoleamide DEA, Linoleamide MEA, Linoleamide MIPA, Linoleic Acid, Linolenic Acid, Maltodextrin, Niacin, Polyacrylate-1 Crosspolymer, Polyester-4, Pyridoxine HCl, Phytosphingosine, Salicylic Acid, Squalane, Squalene, Thiodiglycoamide, Zinc Pyrithione, and mixtures thereof.

The at least one film-former of the disclosed composition varies depending upon the application. These materials can include one or more anionic compounds, non-ionic compounds, amphoteric compounds, zwitterionic compounds, proteins, viscosity modifiers, cationic polymers, anionic polymers, a polyacrylate, a polymethacrylate, a polyacrylate copolymer, a polymethacrylate copolymer, a polyamide, polyaminoamide, polyester, silicone resins, polysaccharides, a silicone fluid, a polyacrylamide, a starch, a gum and mixtures thereof. The at least one film-former be added at a concentration of about 0.05 to about 15 weight % based on the weight of the cosmetic composition. Typically, the at least one film-former is added at a concentration of about 0.1 to about 10 weight % based on the weight of the composition.

Specific examples of the film-formers include polyvinylpyyrolidone, polysilicone 8, polydimethylsiloxane, dimethylsiloxane/3-thiopropyl methyl siloxane copolymer, vinylpyyrolidone/vinylacetate copolymer, polyvinyacetate, starch, cellulose, polyquaternium-4, polyquaternium-11, acrylates/steareth-2 methacrylate crosspolymer, vinylacetate/vinyl neodecanoate copolymer, polyester-5, cetyl ethylhexanoate, vinyl acetate, crotonate/vinyl neodecanoate copolymer, 2-acryamido-2-methyl propane sulfonic acid (AMPS)/acrylic acid (AA) copolymer, AMPS/AA/acryl methacrylate copolymer, polyacrylamide, $C_{13}$-$C_{14}$ isoparaffin, laureth-7, octylacrylamide, acrylate/butylaminoethylmethacrylate copolymer and mixtures thereof.

The at least one hair colorant may be selected from any type of colorant commercially available in the hair coloring industry. This includes direct dyes, oxidative dyesazoic dyes, sulfur dyes, azomethine dyes, triarylmethane, xanthene dyes, phthalocyanin dyes, phenothiazine dyes, pigments, direct dyes, oxidation dyes, nacreous pigments, pearling agents, leuco dyes, optical lightening colorants, natural colorants, optically-variable pigments and mixtures thereof. The at least one hair colorant may be present in an amount from to about 0.001 % to about 5% by weight, based on the weight of the composition.

Specific non-limiting examples of useful dyes are listed in U.S. Patent 7,141,079, U.S. Patent 7,122,062, U.S. Patent 7,094,262, U.S. Patent 7,083,655, U.S. Patent 6,740,130, U.S. Patent 6,139,853 and U.S. Patent 5,951,718, the contents of which are herein incorporated by reference.

The coloring agent may also comprise a colorant chosen from water-soluble or liposoluble dyes or alternatively coloring polymers. The colorant may be present in the composition in content of coloring active material ranging from 0 to 6% (especially 0.01 % to 6%) by weight and typically ranging from 0.01 % to 3% by weight relative to the total weight of the composition.

The liposoluble dyes are, for example, soybean oil, Sudan brown, DC Yellow 11, DC Orange 5, quinoline yellow, Sudan Red III (CTFA name D&C red 17), lutein, quinizarine green (CTFA name DC green 6), Alizurol SS purple (CTFA name DC violet No. 2), DC Blue No. 14, carotenoid derivatives, for instance lycopene, beta-carotene, bixin or capsanthin, annatto, and/or mixtures thereof. The liposoluble dyes, when present, generally have a concentration ranging up to 20% by weight of the total weight of the composition. Typically, when present, the liposoluble dyes are present in an amount from about 0.0001 % to about 6% by weight, based on the weight of the composition.

Among the water-soluble dyes that may be mentioned are dyeing plant extracts such as, for example, *Aleurites moluccana* Willd, *Alkanna tinctoria* Tausch, *Areca catechu L., Arrabidaea chica* E. and B., *Bixa orellana* L (annatto), *Butea monosperma* Lam, *Caesalpina echinata* Lam, *Caesalpina sappan L., Calophyllum inophyllum L., Carthamus tinctorius L., Cassia alata L., Chrozophora tinctoria L., Crocus sativus L., Curcuma longa L., Diospyros gilletii* Wild, *Eclipta prostrata L., Gardenia erubescens* Stapf. and Hutch., *Gardenia terniflora* Schum. and Thonn., *Genipa americana L., Genipa brasiliensis L., Guibourtia demeusei* (Harms) J. Leon, *Haematoxylon campechianum* L., *Helianthus annuus, Humiria balsamifera* (Aubl.) St-Hil., *Isatis tinctoria* L., *Mercurialis perenis, Monascus purpureus, Monascus ruber, Monascus pilosus, Morus nigra L., Picramnia spruceana, Pterocarpus erinaceus* Poir., *Pterocarpus soyauxii* Taub., *Rocella tinctoria* L., *Rothmannia whitfieldii* (Lindl.) Dand., *Schlegelia violacea* (Aubl.) Griseb., *Simira tinctoria* Aublet, *Stereospermum kunthianum* Cham., *Symphonia globulifera* L., *Terminalia catappa L., sorghum, Aronia melanocarpa,* naphthoquinones including lawsone, derived from *Lawsonia inermis* L., also known as henna, or from *Impatiens balsamina,* red wood extracts as described in document WO 98/44902, beetroot juice, the disodium salt of suschin, anthocyans, for instance extracts of red berries, dihydroxyacetone, monocarbonyl or polycarbonyl derivatives such as isatin, alloxan, ninhydrin, glyceraldehyde, mesotartaric aldehyde, pyrazoline-4,5-dione derivatives, and mixtures thereof, these skin-coloring agents optionally being combined with direct dyes or indole derivatives, and/or mixtures thereof.

These dyeing plant extracts may be in the form of a lyophilizate, a paste or a solution: generally, the leaves of the dyeing plant are ground to obtain a powder. This powder is dissolved in an aqueous phase for several hours. The mixture is subsequently centrifuged and then filtered. The filtrate obtained is frozen and then lyophilized.

The nacreous pigments which may be used according to the present invention may be chosen from white nacreous pigments such as mica coated with titanium or with bismuth oxychloride, colored nacreous pigments such as titanium mica with iron oxides, titanium mica with ferric blue or chromium oxide, titanium mica with an organic pigment chosen from those mentioned above, and nacreous pigments based on bismuth oxychloride. The nacreous pigments, if present, may be present in the composition in a concentration ranging up to 50% by weight of the total weight of the composition, such as from 0.1 % to 20%, preferably from 0.1 % to 15%.

The pigments, which may be used according to the present invention, may be chosen from white, colored, inorganic, organic, polymeric, nonpolymeric, coated and uncoated pigments. Representative examples of mineral pigments include titanium dioxide, optionally surface-treated, zirconium oxide, zinc oxide, cerium oxide, iron oxides, chromium oxides, manganese violet, ultramarine blue, chromium hydrate, silica, ferric blue, and mixtures thereof. Representative examples of organic pigments include carbon black, pigments of D & C type, and lakes based on cochineal carmine, barium, strontium, calcium, and aluminum. Other examples of pigments are ultramarines, HC Blue No. 14, Ext. Yellow 7, Yellow 10 Lake, and acid violet 43.

If present, the pigments may be present in the composition in a concentration ranging up to 50 % by weight based on the total weight of the composition, such as from about 0.5% to about 40% by weight, and further such as from about 2% to about 30% by weight.

The coloring agent may also comprise a coloring polymer, i.e. a polymer comprising at least one organic coloring group. The coloring polymer generally contains less than.10% by weight of colorant relative to the total weight of the polymer.

The coloring polymer may be of any chemical nature, especially a polyester, polyamide, polyurethane, polyacrylic, poly(meth)acrylic, polycarbonate, polymers of natural origin, for instance cellulose polymers or chitosan polymers, or mixtures thereof, and preferably polyester or polyurethane polymers.

The coloring polymer may comprise a coloring group [lacuna] may be grafted, especially by covalent bonding, onto the polymer chain, as described in documents WO-A-96/29046, WO-A-92/01022, WO-A-90/07558 and BE-A-609 054.

In particular, the coloring polymer may be a copolymer based on at least two different monomers, at least one of which is an organic coloring monomer.

The monomers of the coloring polymer may be chosen from anthraquinones, methines, bis-methines, aza-methines, arylidenes, 3H-dibenzo[7,i-j]isoquinolines, 2,5-diarylaminoterephthalic acids and esters thereof, phthaloylphenothiazines, phthaloylphenoxazines, phthaloylacridone, anthrapyrimidines, anthrapyrazoles, phthalocyanins, quinophthalones, indophenols, perinones, nitroarylamines, benzodifurans, 2H-1-benzopyran-2-ones, quinophthalones, perylenes, quinacridones, triphenodioxazines, fluoridines, 4-amino-1,8-naphthalimides, thioxanthrones, benzanthrones, indanthrones, indigos, thioindigos, xanthenes, acridines, azines and oxazines.

Coloring monomers are described especially in documents U.S. Pat. No. 4,267,306; U.S. Pat. No. 4,359,570; U.S. Pat. No. 4,403,092, U.S. Pat. No. 4,617,373; U.S. Pat. No. 4,080,355; U.S. Pat. No. 4,740,581; U.S. Pat. No. 4,116,923; U.S. Pat. No. 4,745,173; U.S. Pat. No. 4,804,719; U.S. Pat. No. 5,194,463; U.S. Pat. No. 5,804,719; WO-A-92/07913.

Polymeric colorants are described especially in documents U.S. Pat. No. 4,804,719; U.S. Pat. No. 5,032,670; U.S. Pat. No. 4,999,418; U.S. Pat. No. 5,106,942; U.S. Pat. No. 5,030,708; U.S. Pat. No. 5,102,980; U.S. Pat. No. 5,043,376; U.S. Pat. No. 5,194,463; WO-A-92/07913; WO-A-97/24102, the content of which is incorporated into the present patent application by reference.

Sulphopolyester coloring-polymers such as those described in document WO-A-97/24102 are typically used.

The coloring polymers may be present in the composition according to the disclosure in a content ranging from about 0.01% to about 50% typically ranging from about 0.5% to about 25% by weight and more typically ranging from about 0.2% to about 20% by weight relative to the total weight of the composition.

The at least one hair colorant may also be selected from any type of bleach commercially available in the hair bleaching industry. Bleaching agents include, but not limited to, hydrogen peroxide, perborate and persufate salts such as sodium, potassium, or ammonium salts of perfulfates or perborates. When used, the bleach is present in an amount of from about 0.001% to about 95% by weight, based on the weight of the composition.

The at least one shine agent may be chosen from silicones, alkoxylated silicones, oils, ethoxylated oils, fats, esters, transesters, hydrocarbons, quats and mixtures thereof. The shine agents are present in an amount from about 0.01% to about 95% by weight based on the weight of the composition. Typically, the shine agents are added in an amount from about 1% to about 50 % by weight based on the weight of the composition and more typically from about 5% to about 20% by weight based on the weight of the composition.

Non-limiting examples of shine agents include Amodimethicone, Dimethicone,. Dimethiconol, Cyclomethicone, Phenyltrimethicone, Aminopropyl Phenyltrimethicone, Trimethyl Pentaphenyl Trisiloxane, Cetyl Dimethicone, Alkyl Dimethicone, Potassium Dimethicone PEG-7 Pantheyl Phosphate, Olive oil, Jojoba oil, Apricot oil, Avocado oil, Castor oil, Lanolin, Squalane, Capric/Caprylic Triglyceride, Octyl Palmitate, Isopropyl Palmitate, Isopropyl Myristate, Mineral oil, Petrolatum, Polyquaternium-4, Polyquaternium-11, Behentrimonium Methosulfate, Benetrimonium Chloride and mixtures thereof.

Solvent in the composition is present in an amount from about 10% by weight to about 95% by weight, typically in an amount from about 50% by weight to about 85% by weight and more typically from about 60% by weight to 80% by weight, based on the weight of the composition as a whole. The solvent is typically water, alcohol, glycol or mixtures thereof. Alcohols include ethanol, propanol and butanol. Typically, the alcohol is ethanol or isopropanol. Glycols include hexylene glycol, diethylene glycol, ethylene glycol, propylene glycol, 1,2-butylene glycol, triethylene glycol, dipropylene glycol, and mixtures thereof.

The compositions described above may optionally contain at least one auxiliary ingredient in a positive amount up to about 50% by weight, based on the weight of the composition. The auxiliary ingredient may include proteins, amino acids, conditioners (as described above), cationic conditioners, cationic polymers, nonionic surfactants, anionic surfactants, amphoteric surfactants, zwitterionic surfactants, viscosity modifiers, organosiloxane polymer, waxes, silicone resins, pigments, powders, preservatives, vitamins, antioxidants, alpha hydroxyl acids, beta hydroxyl acids, alpha keto acids, antibacterial agents, sunscreens, preservatives, pH adjusting agents, bleaching agents, perfumes, sequestering agents, anti-dandruff agents and mixtures thereof.

Non-limiting examples of proteins include collagen, deoxyribonuclease, iodized corn protein, milk protein, protease, serum protein, silk, sweet almond protein, wheat germ protein, wheat protein, alpha and beta helix of keratin proteins, hair proteins, such as intermediate filament proteins, high-sulfur proteins, ultrahigh-sulfur proteins, intermediate filament-associated proteins, high-tyrosine proteins, high-glycine tyrosine proteins, tricohyalin, and mixtures thereof.

Non-limiting examples of amino acids include amino acids derived from the hydrolysis of various proteins as well as the salts, esters, and acyl derivatives thereof. Non-limiting examples of such amino acid agents include amphoteric amino acids such as alkylamido alkylamines, i.e. stearyl acetyl glutamate, capryloyl silk amino acid, capryloyl collagen amino acids, capryloyl keratin amino acids, capryloyl pea amino acids, cocodimonium hydroxypropyl silk amino acids, corn gluten amino acids, cysteine, glutamic acid, glycine, hair keratin amino acids, amino acids such as asparatic acid, threonine, serine, glutamic acid, proline, glycine, alanine, cystine, valine, methionine, isoleucine, leucine, tyrosine, phenylalanine, cysteic acid, lysine, histidine, arginine, cysteine, tryptophan, citrulline, lysine, silk amino acids, wheat amino acids and mixtures thereof.

Non-limiting examples of cationic conditioners include quaternium-27, behenamidopropyl PG-dimonium chloride, hydroxyethyl tallowdimonium chloride, hexadimethrine chloride, stearalkonium chloride and cetrimonium chloride.

Non-limiting examples of cationic polymers include polyquaternium-4, polyquaternium-6, polyquaternium-7, polyquaternium-10, polyquaternium-11, polyquaternium-16, polyquaternium-22 and polyquaternium-32.

Non-limiting examples of nonionic surfactants includes alkoxylated derivatives of the following: fatty alcohols, alkyl phenols, fatty acids, fatty acid esters and fatty acid amides, wherein the alkyl chain is in the $C_{12-50}$ range, typically in the $C_{16-40}$ range, more typically in the $C_{24}$ to $C_{40}$ range, and having from about 1 to about 110 alkoxy groups. The alkoxy groups are selected from the group consisting of $C_2$-$C_6$ oxides and their mixtures, with ethylene oxide, propylene oxide, and their mixtures being the typical alkoxides. The alkyl chain may be linear, branched, saturated, or unsaturated. Of these alkoxylated non-ionic surfactants, the alkoxylated alcohols are typical, and the ethoxylated alcohols and propoxylated alcohols are more typical. The alkoxylated alcohols may be used alone or in mixtures with those alkoxylated materials disclosed herein-above.

Other representative examples of such ethoxylated fatty alcohols include laureth-3 (a lauryl ethoxylate having an average degree of ethoxylation of 3), laureth-23 (a lauryl ethoxylate having an average degree of ethoxylation of 23), ceteth-10 (a cetyl alcohol ethoxylate having an average degree of ethoxylation of 10), steareth-10 (a stearyl alcohol ethoxylate having an average degree of ethoxylation of 10), steareth-2 (a stearyl alcohol ethoxylate having an average degree of ethoxylation of 2), steareth-100 (a stearyl alcohol ethoxylate having an average degree of ethoxylation of 100), beheneth-5 (a behenyl alcohol ethoxylate having an average degree of ethoxylation of 5), beheneth-10 (a behenyl alcohol ethoxylate having an average degree of ethoxylation of 10), and other derivatives and mixtures of the preceding.

Commercially available nonionic surfactants are Brij® nonionic surfactants from Uniqema, Willmington, DE. Typically, Brij® is the condensation products of aliphatic alcohols with from about 1 to about 54 moles of ethylene oxide, the alkyl chain of the alcohol being typically a linear chain and having from about 8 to about 22 carbon atoms, for example, Brij 72 (i.e., Steareth-2) and Brij 76 (i.e., Steareth-10).

Also useful herein as nonionic surfactants are alkyl glycosides, which are the condensation products of long chain alcohols, which are the condensation products of long chain alcohols, e.g. $C_8$-$C_{30}$ alcohols, with sugar or starch polymers. These compounds can be represented by the formula (S)n --O--R wherein S is a sugar moiety such as glucose, fructose, mannose, galactose, and the like; n is an integer of from about 1 to about 1000, and R is a $C_8$-$C_{30}$ alkyl group. Examples of long chain alcohols from which the alkyl group can be derived include decyl alcohol, cetyl alcohol, stearyl alcohol, lauryl alcohol, myristyl alcohol, oleyl alcohol, and the like. Preferred examples of these surfactants are alkyl polyglucosides wherein S is a glucose moiety, R is a $C_8$-$C_{20}$ alkyl group, and n is an integer of from about 1 to about 9. Commercially available examples of these surfactants include decyl polyglucoside (available as APG® 325 CS) and lauryl polyglucoside (available as APG® 600CS and 625 CS), all the above-identified polyglucosides APG® are available from Cognis, Ambler, Pa. Also useful herein sucrose ester surfactants such as sucrose cocoate and sucrose laurate.

Other nonionic surfactants suitable for use in the present invention are glyceryl esters and polyglyceryl esters, including but not limited to, glyceryl monoesters, typically glyceryl monoesters of $C_{16}$-$C_{22}$ saturated, unsaturated and branched chain fatty acids such as glyceryl oleate, glyceryl monostearate, glyceryl monoisostearate, glyceryl monopalmitate, glyceryl monobehenate, and mixtures thereof, and polyglyceryl esters of $C_{16}$-$C_{22}$ saturated, unsaturated and branched chain fatty acids, such as polyglyceryl-4 isostearate, polyglyceryl-3 oleate, polyglyceryl-2 sesquioleate, triglyceryl diisostearate, diglyceryl monooleate, tetraglyceryl monooleate, and mixtures thereof.

Also useful herein as nonionic surfactants are sorbitan esters. Preferable are sorbitan esters of $C_{16}$-$C_{22}$ saturated, unsaturated and branched chain fatty acids. Because of the manner in which they are typically manufactured, these sorbitan esters usually comprise mixtures of mono-, di-, tri-, etc. esters. Representative examples of suitable sorbitan esters include sorbitan monooleate (e.g., SPAN® 80), sorbitan sesquioleate (e.g., Arlacel® 83 from Uniqema, Wilmington, Del.), sorbitan monoisostearate (e.g., CRILL® 6 from Croda, Inc., Edison, N.J.), sorbitan stearates (e.g., SPAN® 60), sorbitan trioleate (e.g., SPAN® 85), sorbitan tristearate (e.g., SPAN® 65), sorbitan dipalmitates (e.g., SPAN® 40), and sorbitan isostearate. Sorbitan monoisostearate and sorbitan sesquioleate are particularly preferred emulsifiers for use in the present invention.

Also suitable for use as nonionic surfactants are alkoxylated derivatives of glyceryl esters, sorbitan esters, and alkyl polyglycosides, wherein the alkoxy groups is selected from the group consisting of $C_2$-$C_6$ oxides and their mixtures, with ethoxylated or propoxylated derivatives of these materials being typical. Non-limiting examples of commercially available ethoxylated materials include TWEEN® (ethoxylated sorbitan mono-, di- and/or tri-esters of $C_{12}$ to $C_{18}$ fatty acids with an average degree of ethoxylation of from about 2 to 20).

Non-limiting examples of anionic surfactants include compounds in the classes known as alkyl sulfates, alkyl ether sulfates, alkyl sulfonates, alkyl ether sulfonates, sulfate esters of an alkylphenoxy polyoxyethylene ethanol, alpha-olefin sulfonates, beta alkyloxy alkene sulfonates, alkyl arylsulfonates, alkyl carbonates, alkyl ether carboxylates, fatty acids, succinamates, sulfosuccinates, sarcosinates, octoxynol or nonoxynol phosphates, taurates, fatty taurides, sulfated monoglycerides, fatty acid amino polyoxyethylene sulfates, isothienates and mixtures thereof. Specific examples of anionic surfactants include the ammonium, monoethanolamine, diethanolamine, triethanolamine, isopropylamine, sodium, potassium, lithium, or magnesium salts of lauryl sulfate, dodecylbenzene-sulfonate, lauryl sulfosuccinate, lauryl ether sulfate, lauryl ether carboxylate, lauryl sarcosinate, cocomethyl tauride, and sulfosuccinate half ester amide and mixtures thereof.

Non-limiting examples of amphoteric and zwitterionic surfactants include alkyl, alkyl dimethyl, alkylamido, alkyl amide, alkylamidopropyl, or alkyl dimethylammonium betaine; alkyl amidopropyl or alkyl sulfobetaine; alkyl, alkylampho, or alkylamphocarboxy glycinate; alkyl, or alkyl substituted imidazoline mono or dicarboxylate; sodium salts of alkyl mono- or dicarboxylates; alkyl beta amino acids; alkyl amidopropyl, or alkyl ether hydroxysultaine; alkyl amidopropyl dimethyl ammonia acetate; alkyl ampho mono-or diacetate; alkyl, or alkyl ampho, or alkyl imino dipropionate; alkyl amphopropionate; alkyl beta amino propionic acid; alkyl dipropionate; alkyl beta iminodipropionate; branched or n-alkyl dimethylamidopropionate; alkyl carboxylated propionate; alkyl, or methyl alkyl imidazoline; fluorinated alkyl amphoteric mixtures; and/or nonionic surfactants such as, but not limited to, alkyl, alkyl dimethyl, alkyl amidopropylamine, or bis 2-hydroxy ethyl alkyl amine oxides; alkanolamides; alkyl amides; polyoxyethylene glycol (PEG) of monoglycerides, of sorbitan esters, of branched or linear fatty alcohol ethers, of branched or linear fatty acid ethers, of thioethers; alkyl oxoalcohol PEG; PEG fatty esters; polyoxyethlyene glycol/polyoxpropylene glycol block copolymers; alkyl phenol PEG ethers; alkyl polyglucosides, or polysaccharides, polysiloxane polyethoxylene ether and mixtures thereof.

Specific examples include cocamidopropyl betaine, lauramidopropyl betaine, coco/oleamidopropyl betaine, lauryl betaine, coco betaine, oleyl betaine, cocamidopropyl hydroxysultaine, tallowamidopropyl hydroxysultaine, dihydroxyethyl tallow glycinate, disodium cocoamphodiacetate, disodium cocoamphodipropionate and mixtures thereof.

Non-limiting examples of viscosity modifiers include water swellable/soluble cationic polymers from quaternized polysaccharides such as trimethyl ammonium substituted epoxide of hydroxyethyl cellulose, diallyl dimethyl ammonium salts of hydroxyethylcellulose, deacylated chitin or chitosan, dihydroxypropyl chitosan trimonium chloride, hydroxypropltrimethyl ammonium chloride guar, locust bean, or konjac mannan gum; quaternized synthetics such as acrylamide dimethyl diallyl ammonium chloride copolymers, acrylamide/dimethyl diallyl ammonium chloride/acrylic acid terpolymer, quaternized poly (vinyl pyrrolidone/dimethyl amino ethylmethacrylate), poly (vinylpyrrolidone/methacrylamidopropyl trimethylammonium chloride), polyvinyl pyrrolidone/methylvinylimidazolinium chloride or methyl sulfate copolymer, chloroethylether/dimethylaminopropylamine/adipate or azelate terpolymer, vinylpyrrolidone/methacrylamidopropyl trimethylammonium chloride, acrylonitrile/acrylic acid/dimethylpropanediammonium acrylates sulfate terpolymer. Anionic or nonionic polysaccharide polymers such as gum tragacanth, sodium or propylene glycol alginate, kappa-, iota-, or lambda-carrageenan, guar or hydroxyl propyl guar gum, karaya gum, gum Arabic, locust bean gum, konjac mannan gum, gellan, xanthan, succinoglycan or its acidic or enzymatic hydrolysates, sodium carboxymethyl cellulose, methycellulose, hydroxylethylcellulose, hydroxypropylmethylcellulose, and hydroxypropylecellulose; and/or hydrophobically modified anionic, cationic, or nonionic polymers such as, but not limited to, alkyl and/or substituted hydroxyethylcellulose, lauryl dimethyl ammonium substituted epoxide of hydroxyethylcellulose, propoxylated cellulosic, xanthan, succinoglycan, or polygalactomannoses, alkyl methacrylates/crosslinked acrylic acid copolymer and/or acrylonitrile/acrylates block copolymer.

Examples of organosiloxane polymers useful in the disclosure are commercially available from Goldschmidt Corporation under the ABIL tradename. One typical example is cetyl dimethicone copolyol and has the tradename ABIL WE 09 or ABIL WS 08. The cetyl dimethicone copolyol may be used alone or in conjunction with other non-silicone organic emulsifiers. For example, the cetyl dimethicone copolyol may be used in an admixture with other non-silicone organic auxiliary ingredients such a emulsifiers and emollients. For example, the mixtures identified by the C.T.F.A. names cetyl dimethicone copolyol (and) polyglyceryl 4-isostearate (and) hexyl laurate, or cetyl dimethicone copolyol (and) polyglyceryl-3 oleate (and) hexyl laurate both work well. These blends contain approximately 25-50% of each ingredient, for example ABIL WE 09 contains approximately, by weight of the total ABIL composition, 25-50% cetyl dimethicone copolyol, 25-50%, polyglyceryl 4-isostearate, and 25-50% of hexyl laurate which is an emollient or oil.

Another type of organosiloxane polymer suitable for use in the compositions of the disclosure is sold by Union Carbide under the Silwet™ trademark. These compositions are represented by the following generic formulas:

$$(Me_3Si)_y\text{-}2[(OSiMe_2)_x/yO\text{-}PE]_y$$

wherein PE=--$(EO)_m(PO)_nR$
R=lower alkyl or hydrogen
Me=methyl
EO is polyethyleneoxy
PO is polypropyleneoxy
m and n are each independently 1-5000
x and y are each independently 0-5000, and 8
wherein PE=--$CH_2CH_2CH_2O(EO)_m(PO)_nZ$
Z=lower alkyl or hydrogen, and
Me, m, n, x, y, EO and PO are as described above,
[0170]    with the proviso that the molecule contains a lipophilic portion and a hydrophilic portion. Again, the lipophilic portion can be supplied by a sufficient number of methyl groups on the polymer backbone.

Examples of other polymeric organosiloxane surfactants or emulsifiers include amino/polyoxyalkyleneated polydiorganosiloxanes disclosed in U.S. Pat. No. 5,147,578. Also suitable are organosiloxanes sold by Goldschmidt under the ABIL trademark including ABIL B-9806, as well as those sold by Rhone-Poulenc under the Alkasil tradename. Also, organosiloxane polymers sold by Amerchol under the Amersil tradename, including Amersil ME-358, Amersil DMC-287 and Amersil DMC-357 are suitable. Dow Corning surfactants such as Dow Corning 3225C Formulation Aid, Dow Corning 190 Surfactant, Dow Corning 193 Surfactant, Dow Corning Q2-5200, and the like are also suitable. In addition, products sold under the tradename Silwet by Union Carbide, and products sold by Troy Corporation under the Troysol tradename, those sold by Taiwan Surfactant Co. under the tradename Ablusoft, those sold by Hoechst under the tradename Arkophob, are also suitable for use in the disclosure.

The compositions of the disclosure may contain wax at a concentration about 0.1-25%, preferably 0.5-20%, more typically 1-15% by weight based on the total weight of the composition. Suitable waxes have a melting point of 35 to 120°C, and can be animal waxes, plant waxes, mineral waxes, silicone waxes, synthetic waxes, and petroleum waxes. Examples of waxes in accordance with the disclosure include bayberry, beeswax, candelilla, carnauba, ceresin, cetyl esters, hydrogenated jojoba oil, hydrogenated jojoba wax, hydrogenated microcrystalline wax, hydrogenated rice bran wax, japan wax, jojoba butter, jojoba esters, jojoba wax, lanolin wax, microcrystalline wax, mink wax, montan acid wax, montan wax, ouricury wax, ozokerite, paraffin, PEG-6 beeswax, PEG-8 beeswax, rice bran wax, shellac wax, spent grain wax, sulfurized jojoba oil, synthetic beeswax, synthetic candelilla wax, synthetic carnauba wax, synthetic japan wax, synthetic jojoba oil, ethylene homo- or copolymers, stearoxy dimethicone, dimethicone behenate, stearyl dimethicone, and the like, as well synthetic homo- and copolymer waxes such as PVP/eicosene copolymer, PVP/hexadecene copolymer, and the like.

Silicone resins in the compositions of the disclosure may be added at a concentration in a range of about 0.001-20%, typically 0.01-15%, more typically 0.1-10% by weight based on the total weight of the composition. Examples of suitable silicone resins include siloxy silicate polymers having the following general formula:

$$[(RR'R'')_3SO_{1/2}]_x[SiO_2]_y$$

wherein R, R' and R" are each independently a $C_{1-10}$ straight or branched chain alkyl or phenyl, and x and y are such that the ratio of $(RR'R'')_3SiO_{1/2}$ units to $SiO_2$ units is 0.5 to 1 to 1.5 to 1.

Typically R, R' and R" are a $C_{1-6}$ alkyl, and more preferably are methyl and x and y are such that the ratio of $(CH_3)_3SiO_{1/2}$ units to $SiO_2$ units is 0.75 to 1. For example, a trimethylsiloxy silicate containing 2.4 to 2.9 weight percent hydroxyl groups which is formed by the reaction of the sodium salt of silicic acid, chlorotrimethylsilane, and isopropyl alcohol may be used. The manufacture of trimethylsiloxy silicate is set forth in U.S. Pat. Nos. 2,676,182; 3,541,205; and 3,836,437, all of which are hereby incorporated by reference. Trimethylsiloxy silicate as described is available from Dow Corning Corporation under the tradename 2-0749 and 2-0747, which is a blend of about 40-60% volatile silicone and 40-60% trimethylsiloxy silicate. Dow Corning 2-0749 in particular, is a fluid containing about 50% trimethylsiloxy silicate and about 50% cyclomethicone. The fluid has a viscosity of 200-700 centipoise at 25°C, a specific gravity of 1.00 to 1.10 at 25°C, and a refractive index of 1.40-1.41.

Other silicone resins are silicone esters comprising units of the general formula $R_aR^E{}_bSiO_{[4-(a+b)/2]}$ or $R^{13}{}_xR^E{}_ySiO_{1/2}$, wherein R and $R^{13}$ are each independently an organic radical such as alkyl, cycloalkyl, or aryl, or, for example, methyl, ethyl, propyl, hexyl, octyl, decyl, aryl, cyclohexyl, and the like. a is an number ranging from 0 to 3, b is a number ranging from 0 to 3, a+b is a number ranging from 1 to 3, x is a number from 0 to 3, y is a number from 0 to 3 and the sum of x+y is 3, and wherein $R^E$ is a carboxylic ester containing radical. Typical $R^E$ radicals are those wherein the ester group is formed of one or more fatty acid moieities (e.g. of about 6, often about 6 to 30 carbon atoms) and one or more aliphatic alcohol moieities (e.g. of about 10 to 30 carbon atoms). Examples of such acid moieities include those derived from branched-chain fatty acids such as isostearic, or straight chain fatty acids such as behenic. Examples of suitable alcohol moieties include those derived from monohydric or polyhydric alcohols, e.g. normal alkanols such as n-propanol and branched-chain etheralkanols such as (3,3,3-trimethylolpropoxypropane. Typically, the ester subgroup (i.e. the group containing the carboxylic ester) will be linked to the silicon atom by a divalent aliphatic chain that is at least 2 or 3 carbon atoms in length, e.g. an alkylene group or a divalent alkyl ether group. Most typically, that chain will be part of the alcohol moiety, not the acid moiety. More typically, the cross-linked silicone ester can be a liquid or solid at room temperature. The compound may have a waxy feel and a molecular weight of no more than about 100,000 daltons.

Such silicone resins having the above formula are disclosed in U.S. Pat. No. 4,725,658 and U.S. Pat. No. 5,334,737, which are hereby incorporated by reference. These ingredients are commercially available from General Electric under the tradenames SF 1318 and SF 1312, respectively.

Pigments and powder may be added as a auxiliary ingredient at a concentration of about 0.001-35%, typically 0.01-20%, more typically 0.1-10%, by weight based the total weight of the composition. Typically the pigments and powders have a particle size of 0.02 to 200 microns, typically 0.5 to 100 microns. The particulate matter may be colored or non-colored (for example white). Suitable powders include bismuth oxychloride, titanated mica, fumed silica, spherical silica, polymethylmethacrylate, micronized teflon, boron nitride, acrylate copolymers, aluminum silicate, aluminum starch octenylsuccinate, bentonite, calcium silicate, cellulose, chalk, corn starch, diatomaceous earth, fuller's earth, glyceryl starch, hectorite, hydrated silica, kaolin, magnesium aluminum silicate, magnesium trisilicate, maltodextrin, montmorillonite, microcrystalline cellulose, rice starch, silica, talc, mica, titanium dioxide, zinc laurate, zinc myristate, zinc rosinate, alumina, attapulgite, calcium carbonate, calcium silicate, dextran, kaolin, nylon, silica silylate, silk powder, sericite, soy flour, tin oxide, titanium hydroxide, trimagnesium phosphate, walnut shell powder, or mixtures thereof. The above mentioned powders may be surface treated with lecithin, amino acids, mineral oil, silicone oil or various other agents either alone or in combination, which coat the powder surface and render the particles more lipophilic in nature.

The powder component also may comprise various organic and inorganic pigments. The organic pigments are generally various aromatic types including azo, indigoid, triphenylmethane, anthraquinone, and xanthine dyes which are designated as D&C and FD&C blues, browns, greens, oranges, reds, yellows, etc. Organic pigments generally consist of insoluble metallic salts of certified color additives, referred to as the Lakes. Inorganic pigments include iron oxides, ultramarines, chromium, chromium hydroxide colors, and mixtures thereof.

The auxiliary ingredient may contain a mixture of both pigmented and non-pigmented powders. The percentage of pigments used in the powder component will depend on the type of cosmetic being formulated.

The auxiliary ingredient of the disclosure may contain vitamins and/or coenzymes, as well as antioxidants. These may be added at a concentration of about 0.001-10%, typically 0.01-8%, more typically 0.05-5% by weight based on the total weight of the composition. Suitable vitamins include the B vitamins such as thiamine, riboflavin, pyridoxin, and so on, as well as coenzymes such as thiamine pyrophoshate, flavin adenin dinucleotide, folic acid, pyridoxal phosphate, tetrahydrofolic acid, and so on. Also Vitamin A and derivatives thereof are suitable. Examples are Vitamin A palmitate, acetate, or other esters thereof, as well as Vitamin A in the form of beta carotene. Also suitable is Vitamin E and derivatives thereof such as Vitamin E acetate, nicotinate, or other esters thereof. In addition, Vitamins D and K are suitable.

Suitable antioxidants are ingredients which assist in preventing or retarding spoilage. Examples of antioxidants suitable for use in the compositions of the invention are potassium sulfite, sodium bisulfite, sodium erythrobate, sodium metabisulfite, sodium sulfite, propyl gallate, cysteine hydrochloride, butylated hydroxytoluene, butylated hydroxyanisole, and mixtures thereof.

The auxiliary ingredient may include one or more alpha or beta hydroxy acids or alpha ketoacids. Typical ranges are 0.01-20%, more typically 0.1-15%, and even more typical 0.5-10% by weight based on the total composition. Suitable alpha hydroxy acids and alpha ketoacids are disclosed in U.S. Pat. No. 5,091,171, which is hereby incorporated by reference. The general structure of such alpha hydroxy acids may be represented by the following formula:
(Ra)(Rb)C(OH)COOH

wherein Ra and Rb are H, F, Cl, Br, alkyl, aralkyl, or aryl group of saturated, unsaturated, straight or branched chain or cyclic form having 1-10 carbon atoms, and in addition Ra or Rb may carry OH, CHO, COOH and alkoxy groups having 1 to 9 carbon atoms.

The alpha hydroxy acids may exist in the keto acid form, or the ester form. Examples of such alpha hydroxy acids include glycolic acid, malic acid, pyruvic acid, mandelic acid, lactic acid, methyllactic acid, and mixtures thereof.

Also beta hydroxy acids such as salicylic acid, and derivatives thereof may be included in the compositions of the disclosure. In addition, mixtures of the above alpha and beta hydroxyl acids or alpha ketoacids.

Non-limiting examples of antibacterial agents include bacitracin, phenol, benzethonium chloride, erythromycin, neomycin, tetracycline, chlortetracycline and mixtures thereof.

Non-limiting examples of sunscreens include benzophenones, bornelone, butyl paba, cinnamidopropyl trimethyl ammonium chloride, disodium distryrylbiphenyl disulfonate, paba, potassium methoxycinnamate, butyl methoxydibenzoylmethane, octyl methoxycinnamate, oxybenzone, octocrylene, octyl salicylate, phenylbenzimidazole sulfonic acid, ethyl hydroxypropyl aminobenzoate, menthyl anthranilate, aminobenzoic acid, cinoxate, diethanolamine methoxycinnamate, glyceryl aminobenzoate, titanium dioxide, zinc oxide, oxybenzone, Padimate O, red petrolatum, and mixtures thereof.Examples of artificial tanning actives and accelerators useful in the compositions of the disclosure include, but are not limited to, dihydroxyacetaone, tyrosine, tyrosine esters such as ethyl tyrosinate, phospho-DOPA, and mixtures thereof.

Antiperspirant actives may also be included in the compositions of the disclosure. Suitable antiperspirant actives include astringent metallic salts, especially the inorganic and organic salts of aluminum zirconium and zinc, as well as mixtures thereof. Particularly preferred are the aluminum containing and/or zirconium-containing materials or salts, such as aluminum halides, aluminum chlorohydrate, aluminum hydroxyhalides, zirconyl oxyhalides, zirconyl hydroxyhalides, and mixtures thereof.

Examples of useful anti-acne actives of the disclosure include, but are not limited to, the keratolytics such as salicylic acid (o-hydroxybenzoic acid), derivatives of salicylic acid such as 5-octanoyl salicylic acid, and resorcinol; retinoids such as retinoic acid and its derivatives (e.g., cis and trans); sulfur-containing D and L amino acids and their derivatives and salts, particularly their N-acetyl derivatives, a preferred example of which is N-acetyl-L-cysteine; lipoic acid; antibiotics and antimicrobials such as benzoyl peroxide, octopirox, tetracycline, 2,4,4'-trichloro-2'-hydroxy diphenyl ether, 3,4,4'-trichlorobanilide, azelaic acid and its derivatives, phenoxyethanol, phenoxypropanol, phenoxyisopropanol, ethyl acetate, clindamycin and meclocycline; sebostats such as flavonoids; and bile salts such as scymnol sulfate and its derivatives, deoxycholate, and cholate.

Non-limiting examples of preservatives include ethanol, polyvinyl alcohol, phenoxyethanol, benzyl alcohol, methyl paraben, propyl paraben and mixtures thereof.

Non-limiting examples of pH adjusting agents includes potassium acetate, sodium carbonate, sodium hydroxide, phosphoric acid, succinic acid, sodium citrate, citric acid, boric acid, lactic acid, sodium hydrogen carbonate and mixtures thereof.

Bleaching agents include, but not limited to, hydrogen peroxide, perborate and persufate salts. EDTA and other aminocarboxylates may be used as sequestering agents. Anti-dandruff agents such as zinc pyrithione, salicylic acid, climbazole, ketoconazole, sulfur piroctone olamine, selenium sulfide and mixtures thereof may also be used as an auxiliary ingredient.

The following examples are for illustrative purposes only and are not intended to limit the scope of the claims. In the following examples, Lupasol® G-35 is 50% active and Carbopol® aqua CC is 20% active.

**Determination of the Amine Number and the Acid Number**

The measurement of the Acid and the Amine Value is performed through a common acid-base titration in the presence of a color indicator. The method is based on the European and American Pharmacopoeias and Standard ISO 660.

Specifically, the acid value measures the quantity of free acid functions titratable with NaOH using Phenolphthalein as an indicator (the endpoint is determined by the slight pink color that persists for at least 15 seconds), and is reported as milliequivalent of acid per grams (meq/g) of the acid substance.

Similarly, the amine value measures the quantity of amine functions titratable with HCl using Bromophenol Blue as an indicator (the endpoint is determined by the slight blue color that persists for at least 15 seconds), and is reported as milliequivalent of amine per gram (meq/g) of the polyamine.

**General Procedure for Preparing the Composition of the Disclosure Containing the at Least One Polyamine, the at Least One Acid, the at Least One Water-insoluble Ingredient and Solvent**

The at least one acid, at least one water-insoluble ingredient and other optional oil-based ingredients are mixed at a temperature of at least 25°C in a container A. The at least one polyamine, water and other optional ingredients are mixed at a temperature of at least 25°C in a container B. Next the contents of container B is slowly added to container A with high shear mixing. After all of container B is added, other optional ingredients described above are added while mixing at high shear. Mixing continues until a homogeneous mixture is obtained.

For shaping and styling hair, the homogenous mixture described above may then be used applied to hair followed by an application of a mixture containing the film former and subsequent shaping or styling of the hair. Alternatively, the film former may be mixed into the homogeneous mixture above and then applied to hair followed by shaping on styling the hair. In addition, a mixture containing the film-former may be applied to the hair followed by application of the homogeneous mixture above and subsequent shaping or styling of the hair.

**Water-Resistant Properties of Disclosed Composition**

**A. General Test for the Measurement of the Water-Resistance of Disclosed Composition the at Least One Polyamine, the at Least One Acid, the at Least One Water-insoluble Ingredient and Solvent**

The water-resistance of a surface treated with the disclosed composition can be measured using a Contact Angle Measurement System K-12 manufactured by Kruss (Germany). This instrument allows one to calculate the degree of water-resistance of a solid surface when it was pushed in and pulled out of water by measuring the angle formed by the water-solid interface. The low contact angle denotes a low water-resistance (water spreads on the surface), and the high contact angle denotes a high water-resistance (water beads on the surface).

In this test, a microscope cover glass (Fisher brand 12-542A, 18cm x 18 cm x 0.16 mm) was treated with a solution of the disclosed composition (50 g of Isopropanol (IPA) + 10 g of the disclosed composition) by dipping the cover glass to half of its length into the testing solution and allowing it to dry. The treated cover glass is then mounted on the Kruss instrument and the Advancing Contact Angle (Wetting Contact Angle)/Receding Contact Angle (De-wetting Contact Angle) measured using the following parameters:

Measuring Speed: 3 mm/min

Max Immersion Depth: 5 mm

Min Immersion Depth: 0 mm

Sensitivity: 0.01 g

**B. Measurements of Contact Angle for Mixtures Containing Components the at Least One Polyamine, the at Least One Acid, the at Least One Water-insoluble Ingredient and Solvent**

Following the General procedure described in this above, the Contact Angle of the following compositions were measured (n=5) (Table 1-1):

**Table 1-1**

| IPA (%) | Oleic Acid (%) | Lupasol® G35 (%) | Mineral Oil (%) | Procetyl AWS (%) | Amine # : Acid # | Advancing Contact Angle (°) | Receding Contact Angle (°) |
|---|---|---|---|---|---|---|---|
| 98.08 | 1.04 | 0.38 | 0.5 | - | 1:1 | 92.6 ± 0.6 | 70.7 ± 0.6 |
| 99.87 | 0.10 | 0.016 | 0.008 | - | 1:0.2 | 81.9 ± 1.4 | 65.4 ± 1.7 |
| 88.08 | 1.04 | 0.38 | 0.5 | 10 | 1:1 | 74.0 ± 2.2 | 63.9 ± 0.4 |

The data in Table 1-1 show that when the concentration of the ingredients in the disclosed composition is lowered and the Amine number : Acid number is outside the claimed range, both the Advancing Contact Angle and the Receding Contact Angle decrease to below 66 degrees. A decrease in the contact angle can also be seen in a case where additional ingredients such as nonionic surfactant ( Procetyl AWS) are added to the claimed composition. These results demonstrate that not all compositions necessarily have the disclosed contact angle of 66 degrees.

Table 1-2 lists the contact angles on an untreated and a disclosed composition treated glass surface (n = 10):

**Table 1-2**

| Tested Disclosed Compositions | | | | | | Contact Angle (°) | |
|---|---|---|---|---|---|---|---|
| Isostearic Acid (%) | Lupasol® G35 (%) | Ratio of amine number to acid number | Min. Oil (%) | Water (%) | IPA (%) | Advancing | Receding |
| | | | | | 100 | 9.8 ± 2.9 | 6.7 ± 1.6 |
| 0.3 | 0.13 | 1:0.84 | 0.1 | 16.47 | 83.0 | 69.1 ± 1.0 | 66.4 ± 0.3 |
| 1.5 | 0.13 | 1:4.19 | 0.1 | 15.27 | 83.0 | 91.5 ± 1.1 | 73.4 ± 1.8 |
| 5.0 | 1.0 | 1:1.81 | 2.0 | 9.0 | 83.0 | 94.4 ± 1.0 | 73.5 ± 0.6 |

The data demonstrate a significant increase in water-resistance of the glass surface upon treating with the disclosed composition as evidenced by the increase in both the Advancing Contact Angle and the Receding Contact Angle.

**C. Water-Resistance of Hair Treated with the Disclosed Composition the at Least One Polyamine, the at Least One Acid, the at Least One Water-insoluble Ingredient and Solvent**

Bleached hair (from IHIP, New York) was treated with various shampoo formulas containing the disclosed composition six times (1g shampoo/g hair, 1 cycle = 1 minute shampoo, 30 second rinse). The contact angles between water and the single hair fiber (n = 12) were measured. The results are shown in the following Table (Table 1-3):

**Table 1-3**

| Shampoo Containing Disclosed Composition (qs with water) | | | | | | Contact Angle (°) | |
|---|---|---|---|---|---|---|---|
| SLES[1] (%) | Cocamidopropyl Betaine (%) | Fatty Acid (%) | Polyamine (%) | Water-Insoluble Ingredients (%) | Ratio of Amine # to Acid # | Advancing | Receding |
| 7 | 3 | - | - | - | - | 65.61 ± 4.83 | 0.04 ± 0.12 |
| 7 | 3 | Isostearic Acid (0.5) | Lupasol® G35 (0.4) | Min Oil (0.5) | 1:0.91 | 60.39 ± 2.91 | 38.60 ± 4.53 |
| 7 | 3 | Oleic Acid (0.5) | Carbopol® aqua CC) (0.75) | Min Oil (1.0) | 1:1.05 | 55.06 ± 3.77 | 12.84 ± 11.02 |
| 7 | 3 | Oleic Acid (2.5) | Carbopol® aqua CC) (0.75) | Dow Corning® 200 Fluid 60K (0.75) | 1:5.24 | 58.48 ± 3.94 | 35.10 ± 7.43 |
| 1 - sodium laureth sulfate | | | | | | | |

The data in Table 1-3 shows that hair shampooed with the disclosed composition containing shampoo is water-proof as indicated by the increase in the Receding Contact Angle.

**2. Studies on Non-Transfer of Pigments using the Disclosed Composition Containing the at Least One Polyamine, the at Least One Acid, the at Least One Water-insoluble Ingredient and Solvent**

**2.1 Study on Non-Transfer of Pigments in the Disclosed Composition Containing the at Least One Polyamine, the at Least One Acid, the at Least One Water-insoluble Ingredient and Solvent (aqueous)**

An aqueous formula of the disclosed composition containing a pigment (Red 7) was applied onto hair. The hair was blotted with tissue sheets and then the sheets were read with a spectrophotometer to measure the L value and the a value.

The L value is a measure of lightness/darkness. A lower L value indicates that the color is darker and a higher L value indicates the color is lighter. The a value is a measure of redness. A lower a value indicates less redness and a higher a value indicates more redness. In this study, the transfer of pigment onto the white sheet of tissue paper is indicated by a lower L value (darker) and a higher a value (redder).

**Materials:**

Hair: Regular Bleached hair, 5 inches long (excluding the glue strip), 1cm wide (at glue strip), 5 swatches per treatment type.

Test Treatment: Disclosed composition consisting of: Lupasol® G35 8.29%, Oleic Acid 20.71%, Isododecane 30%, and Red 7 10%, in DI Water. (ratio of amine number to acid number is 1:0.92).

Control Treatment: Traditional Soap formula consisting of: MEA (Monoethanolamine) 4.55%, Oleic Acid 20.71%, Isododecane 30%, and Red 7 10%, in DI Water. (ratio of amine number to acid number is 1:0.97).

Konica Minolta® Spectrophotometer

Kimwipe® Tissue Paper (Large)

Procedure:

First, using the spectrophotometer, take baseline reading of the kimwipe tissue paper. Next, apply treatments, (0.5g per gram of hair) and massage in for a minute. Air dry by hanging for 20 minutes. Place the treated hair between two sheets of kimwipes and place 3.5kg weight on top for 1 minute. Measure L and a values of surfaces of kimwipes where the swatches were pressed. Calculate averages and % changes.

Results:

The hair treated with the disclosed composition had significantly less pigment transfer, by t-test, indicated by lower % change of L value and lower % change of a value (Table 2-1). The hair treated with the Traditional soap had significantly more pigment transfer, by t-test, indicated by higher % change of L value and higher % change of a value.

**Table 2-1**

|  | % change of L value | % change of a value |
|---|---|---|
| Traditional soap | 37.44% | 6510.19% |
| Disclosed Composition | 10.29% | 1646.71 % |

**Conclusion:**

The hair treated with disclosed composition had significantly less pigment transfer than the traditional soap treated swatches.

**2.2 Non-Transfer of Pigment Study of The Disclosed Composition Containing the at Least One Polyamine, the at Least One Acid, the at Least One Water-insoluble Ingredient and Solvent (anhydrous)**

**Objective:**

An anhydrous formula of the disclosed composition containing a pigment (Red 7) was applied onto hair. The hair was blotted with tissue sheets and then the sheets were read with a spectrophotometer to measure the L value and the a value.

The L value is a measure of lightness/darkness. A lower L value indicates that the color is darker and a higher L value indicates the color is lighter. The a value is a measure of redness. A lower a value indicates less redness and a higher a value indicates more redness. In this study, the transfer of pigment onto the white sheet of tissue paper is indicated by a lower L value (darker) and a higher a value (redder).

**Materials:**

Hair: Regular Bleached hair, 5 inches long (excluding the glue strip), 1cm wide (at glue strip), 5 swatches per treatment type.

Test Treatment: Disclosed composition consisting of: Isopropanol 54.5%, Carbopol® Aqua CC 15.5%, Isostearic Acid 10%, Dow Corning® 556 10%, and Red 7 10%. (the ratio of amine number to acid number is 1: 1)

Control Treatment: Traditional Soap formula consisting of: Isopropanol 67.83%, MEA (Monoethanolamine) 2.17%, Isostearic Acid 10%, Dow Corning® 556 10%, and Red 7 10%. (the ratio of amine number to acid number is 1:0.97).

Konica Minolta® Spectrophotometer

Kimwipe® Tissue Paper (large)

**Procedure:**

First, using the spectrophotometer, take baseline reading of kimwipe, next, apply treatments, (0.5g per gram of hair) and massage in for a minute. Air dry by hanging for 20 minutes. Place the treated hair between two sheets of kimwipes and place 3.5kg weight on top for 1 minute. Measure L and a values of surfaces of kimwipes where the swatches were pressed. Calculate averages and % changes.

**Results:**

The hair treated with the disclosed composition had significantly less pigment transfer, by t-test, indicated by lower % change of L value and lower % change of a value (Table 2-2). The hair treated with the anhydrous Traditional soap had significantly more pigment transfer, by t-test, indicated by higher % change of L value and higher % change of a value.

**Table 2-2**

|  | % change of L value | % change of a value |
|---|---|---|
| Traditional soap | 39.75% | 4810.93% |
| Disclosed Composition | 7.38% | 605.95% |

**Conclusion:**

The hair treated with the disclosed composition had significantly less pigment transfer than the traditional soap treated swatches.

**3. Non-Transfer of oil studies with the disclosed composition Containing the at Least One Polyamine, the at Least One Acid, the at Least One Water-insoluble Ingredient and Solvent**

**3.1 Non-Transfer of Oil Study with the Disclosed Composition Containing the at Least One Polyamine, the at Least One Acid, the at Least One Water-insoluble Ingredient and Solvent (aqueous)**

An aqueous formula of the disclosed composition containing mineral oil was applied on the hair. The hair was blotted with oil blotting sheets and then the sheets were read with a spectrophotometer to measure the L value.

**Background:**

The L value is a measure of lightness/darkness. A lower L value indicates that the color is darker and a higher L value indicates the color is lighter. In this study, oil that transfers onto the blotting sheet with a black background will appear dark on the sheets, as indicated by lower L values.

**Materials:**

Hair: Regular Bleached hair, 5 inches long (excluding the glue strip), 1 inch wide (at glue strip), 5 swatches per treatment type.

Test Treatment: Disclosed composition formula consisting of: Isododecane 24.5%, Carbopol® Aqua CC 15.5%, Isostearic Acid 10%, and Mineral Oil 10% in DI Water. (the ratio of the amine number to the acid number is 1:1).

Control Treatment: Traditional Soap formula consisting of: Isododecane 37.83%, MEA 2.17%, Isostearic Acid 10%, and Mineral Oil 10% in DI Water. (The ratio of amine number to the acid number is 1:0.97).

Konica Minolta® Spectrophotometer

Blow Dryer

Johnson and Johnson Clean and Clear® Oil Blotting Sheets

**Procedure:**

First, using the spectrophotometer, take baseline reading of the oil blotting sheets. Next, apply treatments, (0.5g per gram of hair) and massage in for a minute. Blow-dry the swatches at 55°C for 15 minutes. Place the treated hair between two oil blotting sheets and place 3.5kg weight on top for 1 minute. Place the two blotting sheets on a black surface and measure L and a values of surfaces where the swatches were pressed. Calculate averages and % changes.

**Results:**

The swatches treated with the disclosed composition had significant less transfer of oil, by t-test, indicated by low percent change of L value (Table 3-1). The swatches treated with the aqueous traditional soap had significant amount of oil transfer, by t-test, indicated by high percent change of L value.

**Table 3-1**

|  | % change of L value |
|---|---|
| Traditional soap | 8.93% |
| Disclosed Composition | 0.56% |

<u>Conclusion:</u>

The hair treated with the disclosed composition had significantly less transfer of oil than the hair treated with an aqueous formula of traditional soap.

<u>**3.2 Non-Transfer of Oil Study with The Disclosed Composition Containing the at Least One Polyamine, the at Least One Acid, the at Least One**</u> Water-insoluble Ingredient and Solvent (Anhydrous)

An anhydrous formula of the disclosed composition containing mineral oil was applied on the hair. The hair was blotted with oil blotting sheets and then the sheets were read with a spectrophotometer to measure the L value.

<u>Background:</u>

The L value is a measure of lightness/darkness. A lower L value indicates that the color is darker and a higher L value indicates the color is lighter. In this study, oil that transfers onto the blotting sheet with a black background will appear dark on the sheets, as indicated by lower L values.

<u>Materials:</u>

Hair: Regular Bleached hair, 5 in long (excluding the glue strip), 1 in wide (at glue strip), 5 swatches per treatment type.

<u>Test Treatment</u>: Disclosed composition consisting of: Isopropanol 66.2%, Lupasol® G35 5.7%, Oleic Acid 14.3%, and Mineral Oil 14.3%. (the ratio of amine number to the acid number is 1:0.93).

<u>Control Treatment</u>: Traditional Soap formula consisting of: Isopropanol 68.8%, MEA 3.1%, Oleic Acid 14.3%, and Mineral Oil 14.3%. (the ratio of amine number to acid number is 1:0.92)

Konica Minolta® Spectrophotometer

Blow Dryer, comb

<u>Procedure:</u>

First, using the spectrophotometer, take baseline reading of the oil blotting sheets. Next, apply treatments, (0.5g per gram of hair) and massage in for a minute. Blow-dry the swatches at 55°C for 15 minutes. Place the treated hair between two oil blotting sheets and place 3.5kg weight on top for 1 minute. Place the two blotting sheets on a black surface and measure L and a values of surfaces where the swatches were pressed. Calculate averages and % changes.

<u>Results:</u>

The swatches treated with the disclosed composition had significantly less transfer of oil, by t-test, indicated by low percent change of L value (Table 3-2). The swatches treated with the anhydrous traditional soap had significant amount of oil transfer, by t-test, indicated by high percent change of L value.

**Table 3-2**

|  | % change of L value |
|---|---|
| Traditional soap | 26.02% |
| Disclosed Composition | 1.42% |

<u>Conclusion:</u>

The hair treated with the anhydrous formula of the disclosed composition had significantly less transfer of oil than the hair treated with an anhydrous formula of traditional soap.

Overall the results from experiments 1-3 illustrated the ability of the disclosed composition to unexpectedly improve the properties of keratinous substrates. Specifically the disclosed composition unexpectedly improves keratinous properties such as water-resistance, non-transfer of pigment and oil.

The exact formulation that provides the unexpected benefits varies with the specific application. Some non-limiting applications and typical fatty acid and polyamine contents for each application are given in Table A.

**Table A - Typical Fatty Acid, Polyamine and Water Insoluble Ingredient Contents (wt%) for Various Applications**

| | Wt% fatty Acid | % Polyamine | Ratio of Amine Number to Acid Number | Water insoluble ingredient |
|---|---|---|---|---|
| Shampoo | 1.25 - 5.0 | 0.1-0.4 | 1:1 - 1:10 | 0.05 - 0.2 |
| Conditioner | 0.5 - 2 | 0.06 - 0.25 | 1:1 - 1:6 | 0.5 - 2 |
| Styler | 2.5 - 10 | 0.03 - 0.13 | 1:3 - 1:20 | 2.0 - 9.0 |
| Tamer | 4.0 - 16 | 0.3 - 1.5 | 1:1 - 1:5 | 6.0 - 26.0 |
| Curl Definer | 3.5 - 14 | 0.12 - 0.5 | 1:1 - 1:15 | 6.0 - 25.0 |
| Finishing Cream | 4.5 - 18 | 0.12 - 0.5 | 1:1 - 1:20 | 2.0 - 8.0 |

**4. High Humidity Anti-Frizz Study of the Disclosed Composition in Shampoo**

**Objective:**

To study the anti-frizz properties of hair shampooed with shampoo containing the disclosed composition.

**Procedure:**

First shampoo Natural Level 6 (Brown) hair with shampoos containing the disclosed compositions (Test A and B) as well as with the control shampoo (Table 4-1). Wind the hair onto pegboards, bake at 50°C for an hour, then equilibrate overnight at room temperature. Remove the swatches from the pegboards and measure the initial area. Then place them into humidity chamber at 90%RH for 1 hour, then measure the area again. Place them back into the humidity chamber for another hour, then measure the area again. Then place the swatches back into the humidity chamber for 2 hours, then measure the final area. Calculate the area of frizz.

**Table 4-1**

| Control | % | Test A | % | Test B | % |
|---|---|---|---|---|---|
| DI Water | 90 | DI Water | 73.0 | DI Water | 64.5 |
| SLES-2 | 7 | SLES-2 | 7 | SLES-2 | 7 |
| Cocamidopropyl Betaine | 3 | Cocamidopropyl Betaine | 3 | Cocamidopropyl Betaine | 3 |
| | | Carbopol® Aqua CC | 3 | Carbopol® Aqua CC | 4.5 |
| | | Oleic Acid | 10 | Oleic Acid | 15 |
| | | Dimethicone | 4 | Dimethicone | 6 |

**Results:**

Hair shampooed with shampoo containing the disclosed composition (Test A and B) had significantly less frizz than the control after 1, 2, and 4 hours (Table 4-2).

Table 4-2

| | Frizz area (inches $^2$) | | | | Ratio of amine number to acid number |
|---|---|---|---|---|---|
| | Hours in humidity chamber | 1 | 2 | 4 | |
| Sample | | | | | - |
| Control | | 32.36 | 41.03 | 44.45 | - |
| Test A | | 13.61 | 17.38 | 22.62 | 1:5.23 |
| Test B | | 9.63 | 9.58 | 13.08 | 1:5.23 |

**5. Durable Wet Combing Study of Hair Treated with the Disclosed Composition**

**Objective:**

To study the wet combability of hair treated with the disclosed composition, then shampooed up to six times.

Procedure:

Comb wet untreated regular bleached hair with Instron tension meter for baseline measurements.

Blow dry hair and apply the following treatments:

**Table 5-1**

| Control | % | Test | % |
|---------|-----|------|------|
| DI Water | 98.5 | DI Water | 86.86 |
| Polymer JR-30M | 1.5 | Polymer JR-30M | 1.5 |
| | | Lupasol G-35 | 1.64 |
| | | Oleic Acid | 5 |
| | | Mineral Oil | 5 |

Measure wet combing with Instron tension meter.

Shampoo hair up to 6 times.

Measure wet coming after 1, 3 and 6 shampoos.

Calculate % change in Energy Break force.

Results:

Hair treated with test formula containing the disclosed composition (Test) had better wet combing than control after shampooing (Table 5-2). (Lower % change in force, the easier to comb)

**Table 5-2**

| | | | | | Ratio of Amine number to Acid number |
|---|---|---|---|---|---|
| Number of Shampoos | 0 | 1 | 3 | 6 | |
| Sample | % Change in Energy Break (g-in) | | | | |
| Control | -58.18 | -14.1 | 42.52 | 128.21 | - |
| Test | -57.12 | -56.26 | -25.43 | 70.96 | 1:1.12 |

**6. Durable Wet Combing Study of the Disclosed Composition in Shampoo**

Objective:

To study the wet combability of hair shampooed with shampoo the disclosed composition, shampooed up to six times.

Procedure:

Comb wet untreated regular bleached hair with Instron tension meter for baseline measurements.

Shampoo the hair once with the following shampoos:

**Table 6-1**

| Control | % | Test | % |
|---------|-----|------|------|
| DI Water | Q.S. | DI Water | Q.S. |
| SLES-2 | 11 | SLES-2 | 11 |
| Cocamidopropyl Betaine | 1.5 | Cocamidopropyl Betaine | 1.5 |
| Polymer JR-30M | 0.15 | Polymer JR-30M | 0.15 |
| Amodimethicone | 2 | Amodimethicone | 2 |
| | | Lupasol G-35 | 0.2 |
| | | Oleic Acid | 2 |

Measure wet combing with Instron tension meter.

Shampoo hair two times (3x total).

Measure wet combing.

Shampoo hair three times (6x total).

Measure wet combing.

Calculate % change in Energy Break force.

Results:

Hair shampooed with test formula containing the disclosed composition (Test) had statistically better wet combing than control up to 6 shampoos (Table 6-2). (Lower % change in force, the easier to comb)

**Table 6-2**

|  |  |  |  | Ratio of Amine number to Acid number |
|---|---|---|---|---|
| Number of Shampoos | 1 | 3 | 6 |  |
| Sample | % Change in Energy break g/in | | |  |
| Control | 15.29 | 59.10 | 94.99 | - |
| Test | -20.54 | -36.26 | -29.16 | 1:3.7 |

Please add something to the effect of the durability (conditioning effect stays after 6 shampoos)

**7. Curl Retention Study using the Disclosed Composition with a Film Former in High Humidity**

**Materials:**

Hair: Regular Bleached hair, (19cm, 1g each) 5 swatches per treatment type.

Test formula: Disclosed composition consisting of: Lupasol® G35 3.68%, Oleic Acid 10%, Isododecane 10%, and PVP K30 6% in DI Water. (The ratio of amine number to acid number is 1:1)

Control formula: PVP K30 6% solution.

Humidity chamber @ 90-95% relative humidity.

Rollers: 5/8" with metal clips, comb.

**Procedure:**

First apply 1g of the above compositions per swatch and roll them onto curlers. Next, bake them in the 50°C oven for 1 hour, then equilibrate at room temperature overnight. Remove hair from the curlers, hang the swatches, and measure initial length. Place the swatches in humidity chamber (90-95%RH) and measure elongation at 1.5 hours and 5 hours. Calculate averages and % curl retention.

**Results:**

The swatches treated with the disclosed composition had statistically higher % curl retention than the swatches treated with the control solution after 1.5 hours and 5 hours in the humidity chamber (Table 7-1).

**Table 7-1**

|  | % Curl Retention | |
|---|---|---|
|  | 1.5 hours | 5 hours |
| **Control (PVP K30 6% solution only swatches)** | 22.56% | 11.76% |
| **Disclosed Composition** | 37% | 24.11% |

**8. Durable Curl Retention Study using the Disclosed Composition with a Film Former in High Humidity after one shampooing**

**Materials:**

Hair: Regular Bleached hair, (19cm, 1g each) 5 swatches per treatment type.

Test formula: Disclosed composition consisting of: Lupasol® G35 3.68%, Oleic Acid 10%, Isododecane 10%, and PVP K90 6% in DI Water. (the ratio of the amine number to acid number is 1: 1).

Control formula: PVP 6% solution.

Shampoo: 10% SLES-2 shampoo, pH=6.0

Humidity chamber @90-95% relative humidity

Rollers: 5/8" with metal clips, comb.

**Procedure:**

First apply 1g of the above compositions per swatch. Next, shampoo the treated swatches once with the 10% SLES-2 shampoo (wash for 15 seconds and rinse for 10 seconds). Then roll them onto curlers and bake them in the 50°C oven for 1 hour. Then equilibrate at room temperature overnight. Remove hair from the curlers, hang the swatches, and measure initial length. Place the swatches in humidity chamber (90-95%RH) and measure elongation at 1.5 hours and 5 hours. Calculate averages and % curl retention.

**Results:**

Even after shampooing, the swatches treated with the disclosed composition had statistically higher % curl retention than the test swatches, after 1.5 hours and 5 hours in the humidity chamber (Table 8-1).

**Table 8-1**

|  | % Curl Retention | |
|---|---|---|
|  | 1.5 hours | 5 hours |
| **Control** | 25.77% | 10.20% |
| **Test** | 37.36% | 19.12% |

Overall the curl retention studies illustrate the ability of the disclosed composition to unexpectedly improve the shaping and styling properties of hair. Specifically the disclosed composition unexpectedly improves hair properties such as curl retention in hair.

**9. Color Retention Study of the Disclosed Composition with Chromatic Dye**

**Objective:**

To study the durability of hair color by adding the disclosed composition into chromatic dye, using a spectrophotometer.

**Procedure:**

First dye_platinum bleached hair with the disclosed compositions (Test A-D from Table 9-1) mixed into Prizms® Zappy Red (Chromatic Dye). Control swatches were dyed with the Prizms® Zappy Red only. Measure color with spectrophotometer. Shampoo the swatches six times and blow-dry. Measure color.

**Table 9-1**

| Test A | % | Test B | % | Test C | % | Test D | % |
|---|---|---|---|---|---|---|---|
| DI Water | 66 | DI Water | 25 | DI Water | 10.7 | DI Water | 38 |
| Lupasol® G35 | 4 | Carbopol® Aqua CC | 20 | Carbopol® Aqua CC | 34.3 | Amodimethicone | 22 |
| Isostearic Acid | 20 | Isostearic Acid | 25 | Linoleic Acid | 25 | Isostearic Acid | 10 |
| Mineral Oil | 10 | Mineral Oil | 30 | Mineral Oil | 30 | Isopropyl Palmitate | 30 |

**Results:**

Hair dyed with the dye containing disclosed composition (Test A-D) had significantly better color retention than the control, after 6 shampoos (Lower the L value the darker the color) as shown in Table 9-2.

**Table 9-2**

| Sample | L Value | Ratio of amine number to acid number |
|---|---|---|
| Control | 38.9 |  |
| A | 32.85 | 1:1.80 |
| B | 29.87 | 1:1.93 |
| C | 30.19 | 1:1.14 |
| D | 31.63 | 1:0.98 |

**10. Color Retention Study of the Disclosed Composition as a post treatment on hair treated with a chromatic dye**

**Objective:**

To study the color retention effect of the disclosed composition as a post treatment on hair dyed with chromatic dye using a spectrophotometer.

**Procedure:**

First Dye platinum bleached hair with Redken® Hi Fusion R (ChromaticDye). Take baseline readings with a spectrophotometer. Apply the disclosed compositions (Test A-C from Table 10-1). Control remains untreated. Shampoo the swatches six times and blow-dry. Measure color and calculate % ΔE.

**Table 10-1**

| Test A | % | Test B | % | Test C | % |
|---|---|---|---|---|---|
| DI Water | 76 | DI Water | 25 | DI Water | 25 |
| Lupasol® G35 | 4 | Carbopol® Aqua CC | 20 | Carbopol® Aqua CC | 20 |
| Oleic Acid | 10 | Isostearic Acid | 25 | Isostearic Acid | 25 |
| Mineral Oil | 10 | Olive Oil | 30 | Mineral Oil | 30 |

Results:
Hair dyed with chromatic dye that was post treated with the disclosed composition (Test A-C) had significantly better color retention than the untreated control, after 6 shampoos (Lower the Δ E value the darker the color) as shown in Table 10-2.

**Table 10-2**

| Sample | % ΔE value | Ratio of amine number to acid number |
|---|---|---|
| Control | 19.87 | - |
| A | 9.94 | 1:0.92 |
| B | 17.78 | 1:1.93 |
| C | 16.27 | 1:1.93 |

**11. Color Retention Study of The Disclosed Composition as a post treatment on hair treated with an oxidative dye**
Objective:
To study the color retention effect of the disclosed composition as a post treatment on hair dyed with oxidative dye using a spectrophotometer.
Procedure:
First dye platinum bleached Redken® Color Fusion 5VR (Oxidative Dye). Take baseline readings with a spectrophotometer. Apply the disclosed compositions (Test A and B from Table 11-1). Control remains untreated. Shampoo the swatches three times and blow-dry. Measure color and calculate % ΔE.

**Table 11-1**

| Test A | % | Test B | % |
|---|---|---|---|
| DI Water | 15 | DI Water | 55 |
| Carbopol® Aqua CC | 15 | Carbopol® Aqua CC | 15 |
| Oleic Acid | 50 | Oleic Acid | 10 |
| Dimethicone | 20 | Mineral Oil | 20 |

Results:
Hair dyed with oxidative dye that was post treated with the disclosed composition (Test A and B) had significantly better color retention than the untreated control, after 3 shampoos (Lower the Δ E value the darker the color) as shown in Table 11-2.

**Table 11-2**

| Sample | % ΔE value | Ratio of amine number to acid number |
|---|---|---|
| Control | 11.42 | |
| Test A | 5.09 | 1:5.23 |
| Test B | 9.09 | 1:1.05 |

**12. Color Retention Study of The Disclosed Composition in Shampoo**

**Objective:**

To study the color retention effect of shampoo containing the disclosed composition on color treated hair (with chromatic dye), using a spectrophotometer.

**Procedure:**

First color platinum bleached hair with Redken® Hi Fusion R (chromatic dye). Measure baseline with a spectrophotometer. Shampoo the swatches six times with test shampoos A-C (Table 12-1), containing the disclosed compositions as well as with the control shampoo. Blow-dry hair and measure color, then calculate % ΔE.

**Table 12-1**

| Control | % | Test A | % | Test B | % | Test C | % |
|---|---|---|---|---|---|---|---|
| DI Water | 90 | DI Water | 88.8 | DI Water | 87.75 | DI Water | 85.75 |
| SLES-2 | 7 | SLES-2 | 7 | SLES-2 | 7 | SLES-2 | 7 |
| Cocamidopropyl Betaine | 3 | Cocamidopropyl Betaine | 3 | Cocamidopropyl Betaine | 3 | Cocamidopropyl Betaine | 3 |
| | | Lupasol® G35 | 0.2 | Carbopol® Aqua CC | 0.75 | Carbopol® Aqua CC | 0.75 |
| | | Acid Mineral | 0.50 | Oleic Acid | 0.5 | Oleic Acid | 2.5 |
| | | Isostearic Oil | 0.5 | Mineral Oil | 1.0 | Dimethicone | 1.0 |

**Results:**

Hair shampooed with shampoo containing the disclosed composition (Test A-C) had significantly better color retention than the control, after 6 shampoos than the control sample (Lower the delta E value the darker the color) as shown in Table 12-2.

**Table 12-2**

| Sample | % ΔE value | Ratio of amine number to acid number |
|---|---|---|
| Control | 26.11 | |
| Test A | 14.82 | 1:0.91 |
| Test B | 12.62 | 1:1.05 |
| Test C | 21.31 | 1:5.24 |

Overall the color retention studies illustrate the ability of the disclosed composition to unexpectedly inhibit color fading in hair. Specifically the disclosed composition unexpectedly improves hair properties such as water-resistance, pigment transfer, oil transfer and hair color retention. These properties lead to improved hair color retention in both dyed and naturally colored hair.

**13. Non-chemical Hair Color Intensifier Study using the Disclosed Composition with Pigment**

**Objective:**

To study the initial intensity of color using the disclosed composition with pigment on bleached hair dyed black by a spectrophotometer.

**Procedure:**

Measure color (baseline) of black hair swatch with spectrophotometer.

Apply 1.8g of products (Table 13-1) on each swatch (0.7g:1g hair). Massage in.

**Table 13-1**

| Control | % | Test | % |
|---|---|---|---|
| DI Water | 67.8 | DI Water | 66.32 |
| Cl. Orange pigment | 10 | Cl. Orange pigment | 10 |
| MEA | 2.2 | Lupasol G35 | 3.68 |

(continued)

| Control | % | Test | % |
|---|---|---|---|
| Oleic Acid | 10 | Oleic Acid | 10 |
| Mineral Oil | 10 | Mineral Oil | 10 |
|  |  | (The ratio of amine number to acid number is 1:1) |  |

Measure final color.

Calculate %ΔE.

Results:

After applying the product, hair treated with the test formula (disclosed composition with pigment) was significantly more intense in color (% ΔE =32.9) than the hair treated with the control formula (% ΔE =21.9) (traditional soap with pigment).

**14. Chemical Hair Color Intensifier Study using the Disclosed Composition in Chromatic Dye**

**Objective:**

To study the color intensifying effect of the disclosed composition mixed into chromatic dye, using a spectrophotometer.

Procedure:

Platinum bleached hair was colored with: (1) the disclosed compositions (Test A-D in Table 3-1) mixed into Prizms® Zappy Red containing Chromatic Dyes, and (2) the Prizms® Zappy Red only (Control).

**Table 14-1**

| Test A | % | Test B | % | Test C | % | Test D | % |
|---|---|---|---|---|---|---|---|
| DI Water | 66 | DI Water | 25 | DI Water | 10.7 | DI Water | 38 |
| Lupasol® G35 | 4 | Carbopol® Aqua CC | 20 | Carbopol® Aqua CC | 34.3 | Amodimethic one | 22 |
| Isostearic Acid | 20 | Isostearic Acid | 25 | Linoleic Acid | 25 | Isostearic Acid | 10 |
| Mineral Oil | 10 | Mineral Oil | 30 | Mineral Oil | 30 | Isopropyl Palmitate | 30 |

Measure color with spectrophotometer.

**Results:**

Hair colored with the dye containing the disclosed composition (Test A-D) was significantly more intense in color than the hair dyed with the dye alone (Control). Table 14-2 shows the L Value of the colored hair swatches (Lower the L value, the darker and more intense the hair color).

**Table 14-2**

| Sample | L Value | Ratio of amine number to acid number |
|---|---|---|
| Control | 27.95 |  |
| A | 19.76 | 1:1.80 |
| B | 17.81 | 1:1.93 |
| C | 18.35 | 1:1.14 |
| D | 18.22 | 1:0.98 |

**15. Study of The Disclosed Composition used as a barrier on hair during the bleaching or highlighting process**

**Objective:**

To study the barrier effect of the disclosed composition when applied to hair before bleaching, measured by spectrophotometer.

**Procedure:**

First take baseline color reading of black Asian hair using a spectrophotometer. Apply treatments as depicted in Table 15-1 (Control is a traditional soap and Test is the disclosed composition with the ratio of the amine number to acid number is 1:0.54). Bleach the treated hair with Matrix® V-lights (using SoLite® 40vol developer) for 20 minutes at 50°C. Shampoo and dry hair. Measure color. Calculate %ΔE.

**Table 15-1**

| Control | % | Test | % |
|---|---|---|---|
| DI Water | 55.975 | DI Water | 20 |
| MEA | 4.025 | Amodimethicone | 40 |
| Isostearic Acid | 10 | Isostearic Acid | 10 |
| Mineral Oil | 30 | Mineral Oil | 30 |

Results:

Hair pre-treated with the disclosed composition had significantly darker color (% ΔE=5.61) than the hair pre-treated with traditional soap (% ΔE=29.97), i.e., the lower the Δ E value, the darker the color. Therefore the bleach penetrated the traditional soap whereas the disclosed composition protected the hair from the bleach, creating highlighting/lowlighting effects.

Overall the results from the hair coloring studies illustrate the ability of the disclosed composition and method to unexpectedly improve the coloring of hair. Specifically the disclosed composition unexpectedly improves hair color intensity, water-resistance, hair highlighting effect, barrier effect during the bleaching process.

## 16. Disclosed Composition in Hair Shine

Bleached blonde hair (from IHIP) was treated with shine-imparting hair products (0.25 g product/ g hair) and left to dry at room temperature. The hair shine was measured by the gonioreflectometer. In this method, a Xenon White light was shined on the hair and the reflection was measured by the gonioreflectometer.

The shine-imparting hair products are:

A. Disclosed composition solution: 70% Isopropyl alcohol, 4.5% Oleic Acid, 1.8% PEI, 6.0% Isododecane, 1.5% Isopropyl Myristate, 16.2% water (ratio of amine number to acid number is 1:0.92)

B. Glass 01 from Redken: >99% Silicones

C. Optimum Oil Therapy™ Shine Booster from SoftSheen-Carson®: >95% Mineral Oil

The non-treated hair was used as the control. The results are shown in Table 2-1 below.

**Table 16-1**

| Treatment | % Increase of Shine from Control |
|---|---|
| Disclosed Composition (A) | 69% |
| Glass 01 (B) | 143% |
| Oil Therapy™ Shine Booster (C) | 157% |

The data indicate that even though the disclosed composition solution contains only 1.5% of the ingredient that imparts shine to hair (Isopropyl Myristate), it gives a significant shine to hair that is comparable to the anhydrous, oil-based commercial products that contain >95% of the active shine materials (silicones and Mineral Oil).

## 17. DISCLOSED COMPOSITION IN MAKE-UP FORMULATIONS

### Example 17.1- Rubbing Resistancy of Blush

A test powder blush and a control powder blush were made as in the formulas below:

| | Test formula | | Control formula |
|---|---|---|---|
| Talc | q.s. | Talc | q.s. |

(continued)

| Test formula | | Control formula | |
|---|---|---|---|
| Red Iron Oxide | 5% | Red Iron Oxide | 5% |
| Zinc Stearate | 3.5% | Zinc Stearate | 3.5% |
| Finsolv TN* | 15% | Finsolv TN* | 15% |
| DI Water | 1.18% | DI Water | --- |
| PEI | 1.82% | PEI | --- |
| Isostearic Acid | 5% | Isostearic Acid | --- |
| *Finsolv TN (INCI: C12-15 Alkyl Benzoate) | | | |

The test formula above contains the disclosed composition.

The procedure for making the test blush formula is as follows: In a blender, add all of the dry ingredients. Mix at high speed for 1 minute. In a side beaker, premix DI water and PEI. In another side beaker, premix Finsolv TN and Isostearic acid. Then combine both premixes with high speed mixing. Add this mixture into the dry ingredients in the blender and blend at high speed for 2 minutes. The control formula is made in one phase, where all of the ingredients are added into a blender and mixed at high speed for 2 minutes.

The blush formulas were evaluated for rubbing resistancy using the following method: First, the substrate of ten circles (diameter 4.5cm) of white Bioskin plate (Beaulax co., LTD, Japan) was prepared. An amount of 0.05g of the control and test blush were applied to five circles each and spread evenly with a finger. Initial L, a, and b values were measured using the Konica Minolta Spectrophotometer. Next, the blush was rubbed by wrapping a Kimwipe® tissue around a finger and swiping across the circle ten times evenly. Final L, a, and b values were measured and ∆E (total change of color) was calculated and tested for significance.

As a result, the average of ∆E values of control and test are 9.40 and 3.96, respectively. Statistically, these values are significant. This indicates that, overall, the control lost color, and the test retained the color. Therefore the test formula containing the disclosed composition improves the resistancy of the color from rubbing.

**Example 17.2 - Rubbing Resistancy of Mascara**

A test mascara and a control mascara were made as in the formulas below:

| Test formula | | Control formula | |
|---|---|---|---|
| DI water | q.s. | DI water | q.s. |
| Black Iron Oxide | 11% | Black Iron Oxide | 11% |
| Beeswax | 10.5% | Beeswax | 10.5% |
| Ozokerite | 7.9% | Ozokerite | 7.9% |
| Glyceryl Stearate | 5% | Glyceryl Stearate | 5% |
| Stearic Acid | 3% | Stearic Acid | 3% |
| Oleic Acid | 10% | Oleic Acid | --- |
| PEI | 3.63% | PEI | ---- |
| TEA | 1.5% | TEA | 1.5% |
| Sorbitan Sesquioleate | 1% | Sorbitan Sesquioleate | 1% |
| Hydrolyzed Corn Starch | 1% | Hydrolyzed Corn Starch | 1% |
| Hydroxyethylcellulose | 0.2% | Hydroxyethylcellulose | 0.2% |
| Simethicone | 0.1% | Simethicone | 0.1% |

The test formula above contains the disclosed composition.

Both of the formulas are made in two phases, the oil phase and the water phase. Combine both phases at 90°C and mix at high speed. When uniform, pour at 90°C and cool to RT.

The mascara formulas were evaluated for rubbing resistancy using the following method: First, the substrate of six circles (diameter 4.5cm) of white Bioskin plate (Beaulax co., LTD, Japan) was prepared. An amount of 0.05g of the control and test mascaras were applied to three circles each and spread evenly with a finger. The product was dried at ambient conditions for 5 minutes. The initial L, a, and b values were measured using the Konica Minolta Spectrophotometer. Next, the treated circles were rubbed by wrapping a Kimwipe® tissue around a finger and swiping across the circle twice using a clean tissue and the same pressure each time it is swiped. Final L, a, and b values were measured and the average of ΔE value (overall color change) was calculated, then analyzed for statistical significance.

As a result, the average of ΔE value of control and test are **63.62%** and **1.16%,** respectively. Statistically, these values are significant. This indicates that the control had significant color loss overall, and the test retained the color significantly. Therefore the test formula containing the disclosed composition significantly improves the resistance of the mascara from rubbing.

### Example 17.3 - Water Resistancy of Mascara by Dunking

The mascara formulas from example 17.2 were evaluated for water resistancy using the following dunking method: First, the substrate of six circles (diameter 4.5cm) of white Bioskin plate (Beaulax co., LTD, Japan) was prepared. An amount of 0.15g of the control and test mascaras were applied to three circles each and spread evenly with a finger. The products were dried overnight and the initial L, a, and b values were measured using the Konica Minolta Spectrophotometer. Again, another six circles of Bioskin plates were prepared and an amount of 0.15g of the control and test mascaras were applied to three circles each as before. The product was dried at ambient conditions for 1 minute. Next, the treated plates were dunked into DI water for 2 minutes for a total of 54 dunks. The plates were dried at ambient conditions and the final L, a, and b values were measured. The average ΔE value was calculated, then analyzed for statistical significance.

As a result, the average ΔE value of the control and test were **51:15** and 4.08, respectively. Statistically, these values are significant. This indicates that the control had significant color loss overall, and the test retained the color significantly. Therefore the test formula containing the disclosed composition significantly improves the water resistance of the mascara.

### Example 17.4 - Water Resistancy of Mascara by Showering

The mascara formulas from example 17.2 were evaluated for water resistancy using the following showering method: First, the substrate of six circles (diameter 4.5cm) of white Bioskin plate (Beaulax co., LTD, Japan) was prepared. An amount of 0.15g of the control and test mascaras were applied to three circles each and spread evenly with a finger. The products were dried overnight and the initial L, a, and b values were measured using the Konica Minolta Spectrophotometer. Again, another six circles of Bioskin plates were prepared and an amount of 0.15g of the control and test mascaras were applied to three circles each as before. The product was dried at ambient conditions for 1 minute. Next, the treated plates were placed under a shower (80gph, 30°C) for 1 minute. The plates were dried at ambient conditions and the final L, a, and b values were measured. The average ΔE value was calculated, then analyzed for statistical significance.

As a result, the average ΔE value of the control and test were 55.72 and 4.74, respectively. Statistically, these values are significant. This indicates that the control had significant color loss overall, and the test retained the color significantly. Therefore the test formula containing the disclosed composition significantly improves the water resistance of the mascara.

### Example 17.5 - Color Intensity of Lipstick

A. Disclosed composition vs. Control

A set of test and control lipsticks was made as in the formulas below:

| Test A formula | | Control A formula | |
|---|---|---|---|
| Octyldodecanol | q.s. | Octyldodecanol | q.s. |
| Polyethylene | 15% | Polyethylene | 15% |
| Red #7 | 1.5% | Red #7 | 1.5% |
| PEI | 3.68% | PEI | --- |
| Oleic Acid | 10% | Oleic Acid | --- |
| DI Water | 56.32% | DI Water | --- |
| Isododecane | 5% | Isododecane | --- |

The test formula above contains the disclosed composition.

The test formula is made in two phases, water phase and oil phases that are combined at 90°C, then cooled at 6°C for 1hr. The control formula is made in one phase, at 90°C, then cooled at 6°C for 1hr.

The above lipstick formulas were evaluated for initial color intensity using the following method: First, the substrate of ten 2cm x 2cm squares of white Bioskin plate (Beaulax co., LTD, Japan) was prepared. An amount of 0.01 g of the control and test lipsticks were applied to five squares each. The product was spread evenly with a finger and dried overnight at ambient conditions. The L and a values were measured using the Konica Minolta Spectrophotometer and analyzed for statistical significance.

The table below shows the averages of L and a values measured on the Bioskin plate:

|  | L value | a value |
|---|---|---|
| Control A | 55.52 | 53.52 |
| Test A | 52.37 | 62.59 |

The results show that the L value of the test formula is lower than the control, and the a value of the test is higher than the controls. Statistically, these values are significant. This indicates that the test formula, although containing the same amount of pigment as the control formulas, was significantly darker and redder than the control formulas. Therefore, the disclosed composition significantly improves the intensity of the lipstick color.

B. Disclosed composition vs. Control with a Film Former

Another set of test and control lipsticks was made as in the formulas below:

| Test B formula |  | Control B formula |  |
|---|---|---|---|
| Octyldodecanol | q.s. | Octyldodecanol | q.s. |
| Polyethylene | 15% | Polyethylene | 15% |
| Red #7 | 1.5% | Red #7 | 1.5% |
| PEI | 3.68% | PEI | --- |
| Oleic Acid | 10% | Oleic Acid | --- |
| DI Water | 56.32% | DI Water | --- |
| Ganex V220* | --- | Ganex V220* | 5.0% |
| *Ganex V220 (INCI: VP/Eicosene Copolymer) | | | |

The test formula above contains the disclosed composition.

The test formula is made in two phases, water phase and oil phases that are combined at 90°C, then cooled at 6°C for 1hr. The control formula is made in one phase, at 90°C, then cooled at 6°C for 1hr.

The lipstick formulas were evaluated for initial color intensity using the following method: First, the substrate of twelve circles (diameter 4.5cm) of white Bioskin plate (Beaulax co., LTD, Japan) was prepared. An amount of 0.15g of the control and test lipsticks were applied to six circles each. The product was spread evenly with a finger and dried overnight at ambient conditions. The L and a values were measured using the Konica Minolta Spectrophotometer and analyzed for statistical significance.

The table below shows the averages of L and a values measured on the Bioskin plate:

|  | L value | a value |
|---|---|---|
| Control B | 57.19 | 49.59 |
| Test B | 53.18 | 62.56 |

The results show that the L value of the test formula is lower than the controls, and the a value of the test is higher than the controls. Statistically, these values are significant. This indicates that the test formula, although containing the same amount of pigment as the control formulas, was significantly darker and redder than the control formulas. Therefore, the disclosed composition significantly improves the intensity of the lipstick color.

**Example 17.6 - Transfer Resistancy of Lipstick**

A. Disclosed composition vs. Control

The lipstick formulas from example 17.5-A were evaluated for transfer resistancy using the following method: First, the substrate of ten 2cm x 2cm squares of white Bioskin plate (Beaulax co., LTD, Japan) was prepared. An amount of 0.01g of the control and test lipsticks were applied to five squares each. The product was spread evenly with a finger and dried for 1 hour at ambient conditions. Next, a sheet of Kimwipe® tissue was placed on the treated surface and a 3.5kg weight was placed on the top of the tissue for a minute. Then L and a values were measured using the Konica Minolta Spectrophotometer on the tissue, and analyzed for statistical significance.

The table below shows the averages of L and a values measured on the Kimwipe® tissue:

|  | L value | a value |
|---|---|---|
| **Control A** | 72.19 | 27.57 |
| **Test A** | 91.74 | 4.18 |

The results show that the L value of the test formula is higher than the control, and the a value of the test is lower than the control. Statistically, these values are significant. This indicates that the test formula had transferred significantly less color than the control formulas. Therefore, the disclosed composition significantly improves the transfer resistancy of the lipstick.

B. Disclosed composition vs. Control with a Film Former

The lipstick formulas from example 17.5-B were evaluated for transfer resistancy using the following method: First, the substrate of twelve circles (diameter 4.5cm) of white Bioskin plate (Beaulax co., LTD, Japan) was prepared. An amount of 0.15g of the control and test lipsticks were applied to six circles each. The product was spread evenly with a finger and dried for 1 hour at ambient conditions. Next, a sheet of Kimwipe® tissue was placed on the treated surface and a 3.5kg weight was placed on the top of the tissue for a minute. Then L and a values were measured using the Konica Minolta Spectrophotometer on the tissue, and analyzed for statistical significance.

The table below shows the averages of L and a values measured on the Kimwipe® tissue:

|  | L value | a value |
|---|---|---|
| **Control B** | 75.40 | 19.56 |
| **Test B** | 86.79 | 8.86 |

The results show that the L value of the test formula is higher than the controls, and the a value of the test is lower than the controls. Statistically, these values are significant. This indicates that the test formula had transferred significantly less color than the control formulas. Therefore, the disclosed composition significantly improves the transfer resistancy of the lipstick.

**Example 17.7 - Blotting Resistancy of Lipstick**

A. Disclosed composition vs. Control

The lipstick formulas from example 17.5-A were evaluated for blotting resistancy using the following method: First, the substrate of ten 2cm x 2cm squares of white Bioskin plate (Beaulax co., LTD, Japan) was prepared. An amount of 0.01g of the control and test lipsticks were applied to five squares each. The product was spread evenly with a finger and dried for 1 hour at ambient conditions. Next, the lipstick treated surfaces were blotted by placing a sheet of Kimwipe® tissue on the treated surface and placing a 3.5kg weight on top of the tissue for a minute. After one minute, the tissue was peeled off, a new tissue was place on the same place and blotting was repeated again. Blotting was repeated for five times total. The final a values were measured using the Konica Minolta Spectrophotometer on the Bioskin substrate. The average % $\Delta$a was calculated based on the initial a values from example 17.5 and then analyzed for statistical significance. The average % $\Delta$a was obtained using the formula:

$$[(\text{final a value minus initial a value}) / \text{initial a value}] \times 100$$

As a result, the average of % Δa values of the control A and test A were -18.21 and -2.60, respectively. Statistically, these values are significant. The less negative % Δa value indicates that the test formula is significantly darker and redder than the control formula. Therefore, the disclosed composition significantly improves the blotting resistancy of the lipstick.

B. Disclosed composition vs. Control with a Film Former

The lipstick formulas from example 17.5-B were evaluated for blotting resistancy using the following method: First, the substrate of twelve circles (diameter 4.5cm) of white Bioskin plate (Beaulax co., LTD, Japan) was prepared. An amount of 0.15g of the control and test lipsticks were applied to six circles each. The product was spread evenly with a finger and dried for 1 hour at ambient conditions. Next, the lipstick treated surfaces were blotted by placing a sheet of Kimwipe® tissue on the treated surface and placing a 3.5kg weight on top of the tissue for a minute. After one minute, the tissue was peeled off, a new tissue was place on the same place and blotting was repeated again. Blotting was repeated for five times total. The final a values were measured using the Konica Minolta Spectrophotometer on the Bioskin substrate. The average % Δa was calculated based on the initial a values from example V and then analyzed for statistical significance. The average % Δa was obtained using the formula:

$$[(\text{final a value minus initial a value}) / \text{initial a value}] \times 100$$

As a result, the average of % Δa values of the control A and test A were -35.93 and -2.22, respectively. Statistically, these values are significant. The less negative % Δa value indicates that the test formula is significantly darker and redder than the control formula. Therefore, the disclosed composition significantly improves the blotting resistancy of the lipstick.

**Example 17.8 - Fade Resistance of Lipstick**

A. Disclosed composition vs. Control

The lipstick formulas from example 17.5-A were evaluated for fading resistance using the following showering method: First, the substrate of twelve circles (diameter 4.5cm) of white Bioskin plate (Beaulax co., LTD, Japan) was prepared. An amount of 0.15g of the control and test lipsticks were applied to six circles each. The product was spread evenly with a finger and dried for 1 minute at ambient conditions. Initial a values were measured using the Konica Minolta Spectrophotometer. Next, the treated plates were placed under a shower (80gph, 30°C) for 1 minute then dried at ambient conditions. Final a values were measured and the average % Δa was calculated and tested for significance.

As a result, the average of % Δa values of the control A and test A were -15.58 and -7.89, respectively. Statistically, these values are significant. The less negative % Δa value indicates that the test had significantly retained the color, and the test had significant color loss. Therefore the disclosed composition prevents lipstick from fading significantly in the shower.

B. Disclosed composition vs. Control with a Film Former

The lipstick formulas from example 17.5-B were evaluated for fading resistance using the showering method as described the section above.

As a result, the average of % Δa values of the control A and test A were -66.03 and -3.98, respectively. Statistically, these values are significant. The less negative % Δa value indicates that the control had significanty retained the color, and the test had significant color loss. Therefore the disclosed composition prevents lipstick from fading significantly in the shower.

**Example 17.9 - Smear Resistance of Lipstick**

The lipstick formulas from example 17.5-A and 17.5-B were evaluated for smear resistance using the showering method as described in example 17.8. The area of smearing outside the circle was measured using image analysis.

As a result, both of the test lipsticks containing the disclosed composition did not smear at all outside of the circle area of application after showering. Control A lipstick had smeared $7.8cm^2$ outside of the circle. Control B lipstick result was not measurable due to extensive color loss as shown in the example above. Therefore, the disclosed composition makes the lipstick smear resistant.

## 18. Disclosed Composition in Skin Treatment Formulations

### Example 18.1

A test sunscreen crème and a control sunscreen crème were made as in the formulas below:

| Test formula | | Control formula | |
|---|---|---|---|
| DI Water | q.s. | DI Water | q.s. |
| Glycerin | 3.00% | Glycerin | 3.00% |
| Xanthan Gum | 0.20% | Xanthan Gum | 0.20% |
| Cosmedia SP | 0.25% | Cosmedia SP | 0.25% |
| Mexoryl SX | 10.00% | Mexoryl SX | 10.00% |
| PEI | 5.03% | TEA | 7.13% |
| Isostearic Acid | 10.00% | Isostearic Acid | 10.00% |
| Isodecyl Neopentanoate | 10.00% | Isodecyl Neopentanoate | 10.00% |

The procedure for making the test sunscreen formula is as follows: In a main tank, add Isostearic Acid and Isodecyl Neopentanoate and mix at RT. In a side tank, add DI Water. Add slurry of Xanthan Gum, Cosmedia SP, and Glycerin into the water and mix at high speed to thicken. When thickened, add Mexoryl SX and mix at moderate speed. Next, add PEI and mix well. Add the mixture in side tank slowly into the main tank with high speed mixing and side sweeping.

The procedure for making the control sunscreen formula is as follows: In a main tank, add Isostearic Acid and Isodecyl Neopentanoate and mix at RT. In a side tank, add DI Water. Add slurry of Glycerin, Xanthan Gum, and Cosmedia SP into the water and mix at high speed to thicken. When thickened, add Mexoryl SX and mix at moderate speed. Add the mixture in side tank slowly into the main tank with high speed mixing. Lastly, add TEA and mix with side sweep.

The sunscreen formulas were evaluated for water resistancy using the following method: First, the substrate of two 2cm x 2cm squares of white Bioskin plates (Beaulax co., LTD, Japan) was prepared. An amount of 0.02g of the control product was spread evenly on one plate, and 0.02g of the test product was spread evenly on the other plate. Both plates were dried for 5 minutes at ambient conditions, then each were immersed into 100ml of DI water in a jar. The jars containing the treated plates were shaken at speed 6 for up to 2 hours, and the UV absorbance of Mexoryl SX (344nm) in the water solutions was measured over the course of 2 hours. The amount of Mexoryl SX that leached out was calculated, and then the % leached out was calculated.

The table below shows the % of Mexoryl SX that leached out over the course of 2 hours:

| | 5 min | 15 min | 30 min | 60 min | 90 min | 120 min |
|---|---|---|---|---|---|---|
| Control | 65% | 85% | 95% | 100% | 100% | 100% |
| Test | 0% | 6% | 6% | 6% | 8% | 10% |

As a result, the % of Mexoryl SX that leached out into water for the control formula is 100% and for the test formula is 10% after 2 hours of immersion with shaking in water. The results indicate that substantial amount of sunscreen in the control leached out into the water compared to the test. Therefore the disclosed composition improves the water resistancy of the sunscreen.

### Example 18.2

A test anti-perspirant crème and a control anti-perspirant crème were made as in the formulas below:

| Test formula | | Control formula | |
|---|---|---|---|
| DI Water | q.s. | DI Water | q.s. |

(continued)

| Test formula | | Control formula | |
|---|---|---|---|
| REACH AZP-908 Superultrafine* | 16.00% | REACH AZP-908 Superultrafine* | 16.00% |
| PEI | 3.63% | TEA | 5.15% |
| Isostearic Acid | 20.00% | Isostearic Acid | 20.00% |
| Isodecyl Neopentanoate | 10.00% | Isodecyl Neopentanoate | 10.00% |
| *INCI: Aluminum Zirconium Tetrachlorohydrex Gly (15% Aluminum) | | | |

The procedure for making the control anti-perspirant formula above is as follows: In a main tank, add Isostearic Acid and Isodecyl Neopentanoate and mix at RT. In a side tank, add DI Water and Aluminum Zirconium Tetrahydrex Gly, the active ingredient of the anti-perspirant, and mix at RT. Add into main tank slowly with high speed mixing. Lastly, add TEA and mix at moderate speed.

The process for making the Test formula is as follows: In a main tank, add Isostearic Acid and Isodecyl Neopentanoate and mix at RT. In a side tank, add half the amount of the DI Water and PEI and mix at RT. Add into the main tank slowly and mix with high speed mixing. In another side tank, add the remainder of water and Aluminum Zirconium Tetrahydrex Gly and mix at RT. Add into main tank slowly and mix at moderate speed.

The anti-perspirant crème formulas were evaluated for water resistancy using the following method: First, the substrate of two 2cm x 2cm squares of white Bioskin plates (Beaulax co., LTD, Japan) was prepared. An amount of 0.02g of the control product was spread evenly on one plate, and 0.02g of the test product was spread evenly on the other plate. Both plates were dried for 5 minutes at ambient conditions, then each were immersed into 100ml of DI water in ajar. The jars containing the treated plates were shaken at speed 6 for 2 hours. The water was tested for Aluminum content using the Aluminum reagent kit (Eriochrome Cyanine R Method) from Hach, in which the absorbance at 535nm represents the amount of Aluminum present in the water solution.

As a result, the % of Aluminum that leached out into water for the control formula is 70% and for the test formula is none. Therefore the disclosed composition improves the water resistance of the anti-perspirant.

The foregoing description illustrates and describes the present disclosure. Additionally, the disclosure shows and describes only the preferred embodiments of the disclosure, but, as mentioned above, it is to be understood that it is capable of changes or modifications within the scope of the concept as expressed herein, commensurate with the above teachings and/or skill or knowledge of the relevant art. The embodiments described hereinabove are further intended to explain best modes known of practicing the invention and to enable others skilled in the art to utilize the disclosure in such, or other, embodiments and with the various modification required by the particular applications or uses disclosed herein. Accordingly, the description is not intended to limit the invention to the form disclosed herein. Also, it is intended that the appended claims be construed to include alternative embodiments.

All publications, patents and patent applications cited in this specification are herein incorporated by reference, and for any and all purposes, as if each individual publication, patent or patent application were specifically and individually indicated to be incorporated by reference. In the case of inconsistencies, the present disclosure will prevail.

**Claims**

1. A composition comprising
   at least one polyamine,
   at least one acid,
   at least one water-insoluble ingredient,
   solvent and
   optionally at least one auxiliary ingredient
   wherein the molar ratio of the amine groups in the at least one polyamine to the acid groups in the at least one acid is from about 1:0.5 to about 1:30 and wherein a mixture of the at least one polyamine, the at least one acid, the at least one water-insoluble ingredient and solvent form a mixture that has a contact angle of at least about 66 degrees on glass.

2. The composition as claimed in claim 1, wherein the composition comprises the at least one auxiliary ingredient and the at least one auxiliary ingredient is selected from the group consisting of an amino acid, a protein, a cationic conditioner, a cationic polymer, a anionic surfactant, a nonionic surfactant, an amphoteric surfactant, a zwitterionic surfactant, a viscosity modifier, an organosiloxane polymer, a wax, a silicone resin, a pigment, a powder, an antioxidant, a vitamin, an alpha hydroxy acid, a beta hydroxy acid, an alpha keto acid, an antibacterial agent, a sunscreen, an artificial tanning agent, an anti-perspirant agent, an anti-acne agent, a preservative, a pH adjusting agent, a bleaching agent, a perfume, a sequestering agent, an anti-dandruff agent and mixtures thereof.

3. The composition as claimed in claims 1 or 2, wherein the at least one polyamine is selected from the group consisting of a polyethyleneimine, a polyvinylamine, an aminated polysaccharide, an amine substituted polyalkylene glycol, an amine substituted polyacarylate, a protein, an amine substituted polyester, a polyamino acid, an amodimethicone, a polyalkylamine, diethylene triamine, triethylenetetramine, spermidine, spermine, aminosilicone and mixtures thereof and/or is selected from the group consisting of a polyethyleneimine, a polyvinylamine, chitosan, polylysine, polyacrylate-1-crosspolymer and mixtures thereof.

4. The composition as claimed in any one of claims 1 to 3, wherein the at least one acid is selected from the group consisting of a fatty carboxylic acid, a fatty ether carboxylic acid, a fatty ether phosphoric acid, a fatty phosphoric acid and mixtures thereof, and/or is a monoacid or a polyacid and/or is selected from the group consisting of capric acid, caprylic acid, isosteric acid, oleic acid, stearic acid, lauric acid, linoleic acid, linolenic acid, laureth-5 carboxylic acid, laureth-11 carboxylic acid, cetyl phosphosphate, stearyl phosphate, oleth-3 phosphate, oleth-10 phosphate and mixtures thereof.

5. The composition as claimed in any one of claims 1 to 4, wherein the at least one water-insoluble ingredient is selected from the group consisting of an oil, a fatty ester, a hydrocarbon oil, a silicone, a wax, a fatty acid, a fatty alcohol and mixtures thereof and/or is selected from the group consisting of olive oil, avocado oil, coconut oil, mineral oil, isopropyl palmitate, capric/caprylic triglyceride, isododecane, polyisobutene, dimethicone, phenyltrimethicone, beeswax and mixtures therefore.

6. The composition as claimed in any one of claims 1 to 5, wherein i) the at least one polyamine is present in a positive amount up to about 30% by weight, based on the weight of the composition; ii) the at least one acid is present in a positive amount up to about 50% by weight, based on the weight of the composition; iii) the at least one water-insoluble ingredient is present in a positive amount up to about 50% by weight, based on the weight of the composition; iv) solvent is present in an amount of from about 10% to about 90% by weight, based on the weight of the composition and/or v) the at least one auxiliary ingredient is present in a positive amount up to about 50%, based on the weight of the composition.

7. A method of treating a keratinous substrate comprising contacting the keratinous substrate with the composition as claimed any one of claims 1 to 6.

8. The method as claimed is claim 7, wherein the keratinous substrate is at least one selected from the group consisting of hair, skin, lips, nails and eyelashes.

9. A composition as claimed in any one of claims 1 to 6 further comprising i) at least one conditioning agent; ii) at least one film former; iii) at least one hair colorant or iv) at least one shine agent.

10. The composition as claimed in claim 9, wherein the at least one conditioning agent is selected from the group consisting of amino acids, proteins, extracts, fats, oils, esters, transesters, hydrocarbons, quats, polyquats, zwitterionic surfactants, amphoteric surfactants, alcohols, polyols, humectants, alkanolamides, fatty acids, ketones, and mixtures thereof and/or is selected from the group consisting of Arginine, Asparagine, Aspartic Acid, Carnitine, Cocoyl sarcosine, Glycine, Glutamic acid, Histidine, Hydroxyproline, Acetyl Hydroxy praline, Isoleucine, Lysine, Lauroyl Lysine, Lauroyl Sarcosine, Methionine, Phenylalanine, Polylysine, Potassium Cocoyl Glutamate, Proline, Sarcosine, Serine , Rice amino acids, Silk amino acids, Wheat amino aids, Sodium Glutamate, Sodium Lauroyl Glutamate, Sodium PCA, Stearoyl sarcosine, Threonine, Tyrosine, Tryptophan, Valine, Casein, Collagen, Procollagen, Gelatin, Keratin, Glycoproteins, Hydrolyzed wheat protein, Hydrolyzed soy protein, Hydrolyzed oat protein, Hydrolyzed rice protein, Hydrolzed vegetable protein, Hydrolyzed yeast protein, Whey protein , Ginkgo Biloba Nut extract, Salix Alba (Willow) Bark Extract, Morus Alba (Mulberry) Leaf, Behentrimonium Chloride, Behenamidopropyl PG-Dimonium Chloride, Behentrimonium Methosulfate, Cocotrimonium Methosulfate, Olealkonium Chloride, Steartrimonium Chloride, Babassuamidopropalkonium Chloride, Hydroxypropyl Guar , Hydroxypropyltrimonium chloride, Laurdimonium Hydroxypropyl Hydrolyzed Soy Protein, Steardimonium Hydroxypropyl Hydrolyzed Wheat Protein, Quaternium-22, Quaternium-27, Quaternium-87, Polyquaternium-4, Polyquaternium-6, Polyquaternium-10, Polyquaternium-11, Polyquaternium-44, Polyquaternium-67, Silicone Quaterium-8, Amodimethicone, Aminopropyldimethicone, Phenyltrimethicone, Cyclomethicone, Dimethicone, Hexyl Dimethicone, Dilinoleamidopropyl Dimthylamine Dimethicone PEG-7 Phosphate, C26-28 Alkyl Dimethicone, PEG-8 Dimethicone, PPG-12 Dimethicone, Polysilicone-13, Trideceth-9 PG-Amodimethicone, Bis-PEG-12 Dimethicone Beeswax, Capric/Caprylic Triglyceride, Petrolatum, Mineral Oil, Lanolin Oil, Cocos nucifera (Coconut) Oil, Olea Europea (Olive) Fruit Oil, Simmondsia Chinensis (Jojoba) Seed Oil, Prunus Armeniaca (Apricot) Kernel Oil, Crambe Abyssinica Seed Oil, Vegetable Oil, Zea Mays (Corn) Oil, Acetylated Lanolin Alcohol, Cetearyl Isononanoate, Cetearyl Ethylhexanoate, Cetearyl Palmitate, Hydrogenated Olive Oil Hexyl Esters, Triethylhexanoin, Ceramide-3, Caprylyl Glycol, Cetyl Glycol, Glycerin, Panthenol, Phytantriol, Methanediol, Inositol, PPG-35-Buteth-45, PPG-5 Butyl Ether, Cocoamidopropyl Betaine, Coco-Betaine, Cocoamidopropyl Hydroxysultaine, Lauramidopropyl Betaine, Lauryl Betaine, Oleamidopropyl Betaine, Disodium Cocoamphodiacetate, Disodium Cocoamphodipropionate, Disodium Lauroamphodiacetate, Sodium Cocoamphopropionate, Sodium Cocoamphoacetate, Acetamide MEA, Behenamide MEA, Linoleamide DEA, Linoleamide MEA, ,Linoleamide MIPA, Linoleic Acid, Linolenic Acid, Maltodextrin, Niacin, Polyacrylate-1 Crosspolymer, Polyester-4, Pyridoxine HCl, Phytosphingosine, Salicylic Acid, Squalane, Squalene, Thiodiglycoamide, Zinc Pyrithione, and mixtures thereof.

**11.** The composition as claimed in claim 9, wherein the at least one film-former is selected from the group consisting of polyvinylpyyrolidone, polysilicone 8, polydimethylsiloxane, dimethylsiloxane/3-thiopropyl methyl siloxane copolymer, vinylpyyrolidone/vinylacetate copolymer, polyvinyacetate, starch, cellulose, polyquaternium-4, polyquaternium-11, acrylates/steareth-2 methacrylate crosspolymer, vinylacetate/vinyl neodecanoate copolymer, polyester-5, cetyl ethylhexanoate, vinyl acetate, crotonate/vinyl neodecanoate copolymer, 2-acryamido-2-methyl propane sulfonic acid (AMPS)/acrylic acid (AA) copolymer, AMPS/AA/acryl methacrylate copolymer, polyacrylamide, $C_{13}$-$C_{14}$ isoparaffm, laureth-7, octylacrylamide, acrylate/butylaminoethylmethacrylate copolymer and mixtures thereof.

**12.** The composition as claimed in claim 9, wherein the at least one hair colorant is selected from the group consisting of a bleaching agent, an oxidative dye, a substantive dye, direct dyes, oxidative dyesazoic dyes, sulfur dyes, azomethine dyes, triarylmethane, xanthene dyes, phthalocyanin dyes, phenothiazine dyes, pigments, coloring polymers, direct dyes, oxidation dyes, nacreous pigments, pearling agents, leuco dyes, optical lightening colorants, natural colorants, optically-variable pigments and mixtures thereof.

**13.** The composition as claimed in claim 9, wherein the at least one shine agent is selected from the group consisting of silicones, alkoxylated silicones, oils, ethoxylated oils, fats, esters, transesters, hydrocarbons, quats and mixtures thereof and/or the at least one shine agent is selected from the group consisting of Amodimethicone, Dimethicone, Dimethiconol, Cyclemethicone, Phenyltrimethicone, Aminopropyl Phenyltrimethicone, Trimethyl Pentaphenyl Trisiloxane, Cetyl Dimethicone, Alkyl Dimethicone, Potassium Dimethicone PEG-7 Pantheyl Phosphate, Olive oil, Jojoba oil, Apricot oil, Avocado oil, Castor oil, Lanolin, Squalane, Capric/Caprylic Triglyceride, Octyl Palmitate, Isopropyl Palmitate, Isopropyl Myristate, Mineral oil, Petrolatum, Polyquaternium-4, Polyquaternium-11, Behentrimonium Methosulfate, Benetrimonium Chloride and mixtures thereof.

**14.** The composition as claimed in any one of claims 9 to 13, wherein the at least one conditioning agent is present in an amount of from about 0.001 % to about 50% by weight, based on the weight of the composition, the at least one film-former is present at a concentration of about 0.05 to about 15 weight % based on the weight of the composition, the at least one hair colorant is present in an amount up from about 0.005 to about 10 % by weight, based on the weight of the composition or the at least one shine agent is present in an amount of from about 0.01% to about 95% by weight based on the weight of the composition.

**15.** The composition as claimed in any one of claims 1 to 6 or 9 to 14 wherein the composition is a mousse, is a gel, is a lotion, is a shampoo, is a styling product, is a hairspray, or is a conditioner and treatment product.

**16.** A method of conditioning hair comprising contacting hair with the composition as claimed in any one of claims 9, 10 or 14 wherein said composition comprises the at least one conditioning agent.

**17.** A method for shaping and styling hair comprising contacting the composition as claimed in any one of claims 9, 11 or 14 to hair wherein said composition comprises the at least one film former.

**18.** The method as claimed in claim 17, wherein applying the composition to hair comprises i) applying a first mixture to hair then applying a second mixture to the hair then shaping or styling the hair, wherein the first mixture comprises the at least one polyamine, the at least one acid, the at least one water-insoluble ingredient and solvent and optionally the at least one auxiliary ingredient and the second mixture comprises the at least one film former and optionally solvent and optionally the at least one auxiliary ingredient, ii) applying a first mixture to hair then applying a second mixture to the hair then shaping or styling the hair, wherein the first mixture comprises the at least one film former and optionally solvent and optionally the at least one auxiliary ingredient and the second mixture comprises the at least one polyamine, the at least one acid, the at least one water-insoluble ingredient and solvent and optionally the at least one auxiliary ingredient or iii) applying a mixture to hair and then shaping or styling the hair wherein the mixture comprises the at least one polyamine, the at least one acid, the at least one water-insoluble ingredient, solvent, the at least one film and optionally the at least one auxiliary ingredient.

19. A method for inhibiting color fading in hair comprising contacting hair with the composition as claimed in any one of claims 1 to 6.

20. The method as claimed in claim 19, wherein the hair is naturally colored hair, is dyed hair, is dyed with a dye and then the composition is applied to the hair or is applied simultaneously with the composition.

21. The method as claimed in claim 20, wherein the dye is at least one dye selected from the group consisting of a bleaching agent, an oxidative dye, a substantive dye, direct dyes, oxidative dyesazoic dyes, sulfur dyes, azomethine dyes, triarylmethane, xanthene dyes, phthalocyanin dyes, phenothiazine dyes, pigments, coloring polymers, direct dyes, oxidation dyes, nacreous pigments, pearling agents, leuco dyes, optical lightening colorants, natural colorants, optically-variable pigments and mixtures therof.

22. A method for coloring hair comprising contacting the composition as claimed in one of claims 9, 12 or 14 with hair, wherein said composition comprises the at least one hair colorant.

23. The method as claimed in claim 22, wherein i) the at least one polyamine, the at least one acid, the at least one water-insoluble ingredient, solvent and optionally the at least one auxiliary ingredient are contacted with hair and the at least one hair colorant is subsequently contacted with the hair; ii) the at least one polyamine, the at least one acid, the at least one water-insoluble ingredient, solvent, optionally the at least one auxiliary ingredient and the at least one hair colorant are simultaneously contacted with the hair, iii) the at least one hair colorant and optionally solvent and the at least one auxiliary ingredient are first contacted with hair and the at least one polyamine, the at least one acid, the at least one water-insoluble ingredient, solvent, optionally the at least one auxiliary ingredient are subsequently contacted with hair or iv) the at least one coloring agent is applied to portions of the hair followed by application of the other components and then other portions of the hair have all components except the at least one coloring agent applied followed by application of the coloring agent.

24. A method of imparting shine onto hair comprising contacting hair with the composition as claimed in any one of claims 9, 13 or 14 wherein said composition comprises the at least one shine agent.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 7141079 B
- US 7122062 B
- US 7094262 B
- US 7083655 B
- US 6740130 B
- US 6139853 A
- US 5951718 A
- WO 9844902 A
- WO 9629046 A
- WO 9201022 A
- WO 9007558 A
- BE 609054 A
- US 4267306 A
- US 4359570 A
- US 4403092 A
- US 4617373 A
- US 4080355 A
- US 4740581 A
- US 4116923 A
- US 4745173 A
- US 4804719 A
- US 5194463 A
- US 5804719 A
- WO 9207913 A
- US 5032670 A
- US 4999418 A
- US 5106942 A
- US 5030708 A
- US 5102980 A
- US 5043376 A
- WO 9724102 A
- US 5147578 A
- US 2676182 A
- US 3541205 A
- US 3836437 A
- US 4725658 A
- US 5334737 A
- US 5091171 A

**Non-patent literature cited in the description**

- International Cosmetic Ingredient Dictionary and Handbook. 2000, vol. 2, 1701-1703
- International Cosmetic Ingredient Dictionary. Cosmetic, Toiletry and Fragrance Association Inc,